# EUROPEAN PATENT APPLICATION

(11) **EP 3 777 893 A1**
(43) Date of publication of application: **17.02.2021**
(21) Application number: 19775597.8
(22) Date of filing: 29.03.2019
(51) Int. Cl.: A61K 47/54, A61K 45/00, C07D 519/00, G01N 33/53

(54) **USE OF BIS-IMINOBIOTIN COMPOUND FOR DRUG DELIVERY PURPOSES**

(30) Priority: 30.03.2018 JP 2018069302
(71) Applicant: Mitsui Chemicals, Inc., Minato-ku Tokyo 105-7122 (JP); The University of Tokyo, Bunkyo-ku, Tokyo 113-8654 (JP); Savid Therapeutics Inc., Tokyo 168-0064 (JP)
(72) Inventor: SUZUKI Tsuneji, Sodegaura-shi, Chiba 299-0265 (JP); MANO Kosuke, Sodegaura-shi, Chiba 299-0265 (JP); TOTANI Yoshiyuki, Sodegaura-shi, Chiba 299-0265 (JP); SHIMIZU Yohei, Sapporo-shi, Hokkaido 060-0808 (JP); SUGIYAMA Akira, Tokyo 113-8654 (JP); TSUKAGOSHI Masanobu, Tokyo 168-0064 (JP)
(74) Representative: Mewburn Ellis LLP
(86) International application number: PCT/JP2019/014284
(87) International publication number: WO 2019/189867

(57) **Abstract**

The technical problem of the present invention is to provide a bis-iminobiotin compound which has a structure that enables the easy bonding of a bis-iminobiotin moiety to a drug or a fluorescent compound and is useful for the delivery of a drug to a streptavidin-labeled substance. The bis-iminobiotin compound that can solve the technical problem of the present invention is represented by general formula 9. (In the formula, A, D and E independently represent a spacer capable of bonding two bicyclo rings to each other, wherein E represents a structure that may be branched, each of A, D and E may have a substituent, and A, D and E may together form a cyclic structure; J represents a functional group for achieving a click reaction; G represents a spacer capable of bonding E to J; and R represents a hydrogen atom, an acetyl group, a benzyl group, a trifluoroacetyl group or a Boc group.)

## Description

### Technical field

The present invention relates to applications for drug delivery of bis-iminobiotin compounds, which are compounds useful in the field of pharmaceuticals.

### Technical background

Biotin (represented by formula 1 below) is one of the moleculars present in vivo and is known to bind tightly to streptavidin (Kd=10⁻¹⁵M) and is widely used in biological studies.

It is known to utilize these strong interactions and to utilize modified biotin and streptavidin with controlled binding force for pharmaceuticals.
Iminobiotin (represented by formula 2 below) is one of the modified biotins, and is a compound in which the cyclic urea structure of biotin becomes a cyclic guanidine. Iminobiotin is used as a structure capable of adjusting the binding force with streptavidin, and further, a bis-iminobiotin compound represented by the general formula 3 is known as a structure available for a pre-targeting medicine (Patent Document 1)

To date, there has been reported a side chain W which is bonded to a spacer V of a bis-iminobiotin compound represented by general formula 3 having a polyethylene glycol or an amide bond, and at its terminal, an amine or a carboxylic acid for binding a compound as an active ingredient of a fluorescent compound or a drug. However, it was necessary to protect the highly reactive functional groups and deprotect them later when attaching a fluorescent compound or the like to these ends.

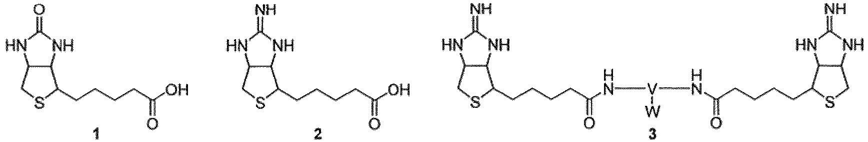

For example, a bis-iminobiotin compound represented by the following formula 4 and formula 5 described in Patent Document 1 has a fluorophore or a drug in a molecule, and is reported to be useful in drug delivery and diagnostic techniques based on a pretargeting method. However, the synthesis of these molecules requires multiple steps, and the selective modification of functional groups and the desorption of protecting groups at the end of the synthesis may be problematic.

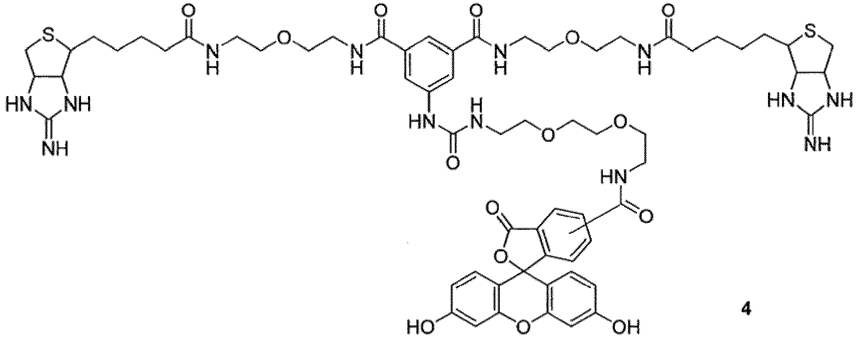

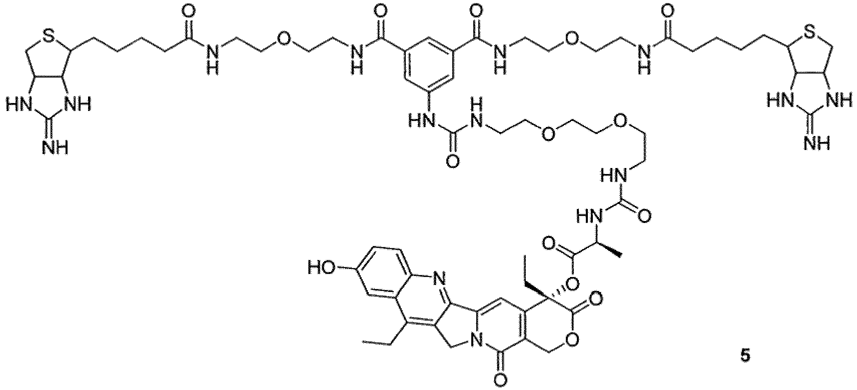

Therefore, it was necessary to develop a compound in which the bis-iminobiotin moiety is common and only the terminal can be conveniently converted. Therefore, the synthesis of the bis-iminobiotin compound became multi-step, and there was room for improvement from the viewpoint of the industrial production, and the technique of combining the biotin part with the drug or the fluorescent compound more easily was required.

### Prior-art document

### Patent Application

[Patent Document 1] WO 2015/125820

### Summary of the invention

### Problems to be solved by the present invention

However, a method for conveniently binding a drug or a fluorescent compound with a bis-iminobiotin moiety has not been disclosed in the prior art at present.

It is an object of the present invention to provide a bioiminobiotin compound useful for drug delivery to a substance labeled with streptavidin, which has a structure for conveniently binding a drug or a fluorescent compound with a bioiminobiotin moiety.

### Means for solving the problems

In order to solve the problem of the present invention, the present inventors have devised a molecular design utilizing a click reaction defined by K. B. Sharpless.

Among the click reactions, Huisgene cyclization, in which an alkyne and an azide group are reacted to form a triazole, is one of the most used reactions. By synthesizing a compound having an alkyne or an azide group at the bis-iminobiotin terminal, it is considered that it becomes possible to conveniently modify the terminal of the side chain without the desorption of a complicated protecting group.

The present inventor has completed the present invention by obtaining a molecular structure of a bis-iminobiotin compound according to the present invention based on the above points.

The bisimino biotin compound according to the present invention is characterized in that it is represented by the following general formula 9

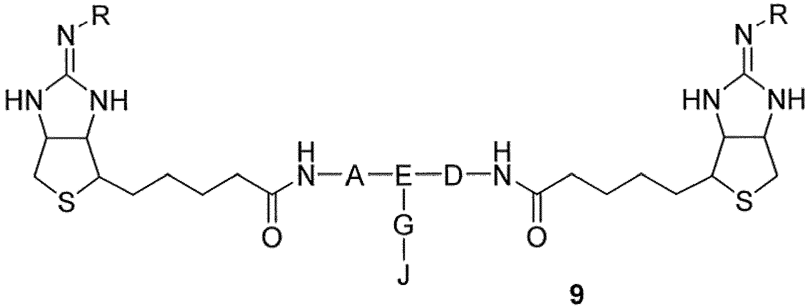

(In the formula, A, D, and E are spacers connecting two bicyclo rings, and E represents a structure capable of taking a branch, and may each have a substituent, and may form a ring structure. J represents a functional group for performing a click reaction, G represents a spacer for bonding E and J, and R represents a hydrogen, an acetyl group, a benzyl group, a trifluoroacetyl group or a tertiary butoxycarbonyl [hereinafter, Boc] group.)

However, the following compounds 10-24 and 10-25 are removed from the above-mentioned bis-iminobiotin compound shown by the general formula 9

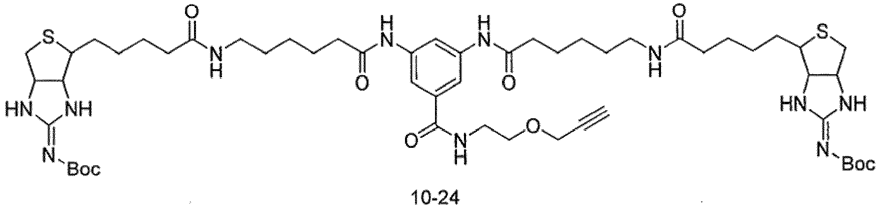

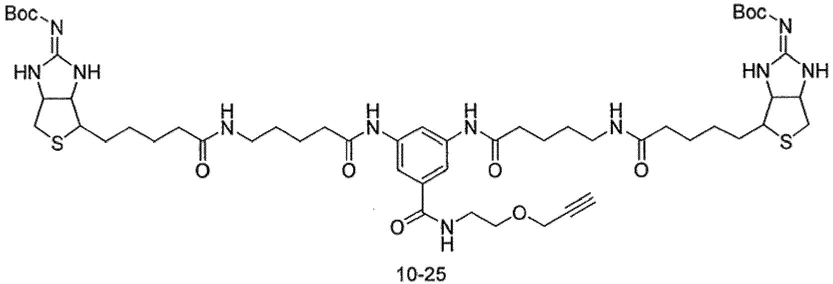

The bis-iminobiotin compound represented by the above general formula 9 is useful as a substrate for drug delivery to a substance labeled with streptavidin.

A drug-conjugated bioiminobiotin compound obtained by binding a drug to a bioiminobiotin compound represented by the above general formula 9 can be used as a drug delivery agent to a substance labeled with streptavidin.

A method of delivering a drug to a streptavidin-labeled substance using a bis-iminobiotin compound represented by the above general formula 9 comprises binding a drug to a substrate for drug delivery, binding a substrate for drug delivery to said streptavidin-labeled substance, and binding said drug to said streptavidin-labeled substance via said substrate for drug delivery,
wherein the aforementioned base material for drug delivery is a bis-iminobiotin compound represented by the above-mentioned general formula 9.

The bis-iminobiotin compound represented by the above general formula 9 can be used as a substrate for drug delivery in a method for producing a drug delivery agent for drug delivery to a substance labeled with streptavidin.

A method for producing a drug delivery agent for drug delivery to a substance labeled with streptavidin using a bis-iminobiotin compound represented by the above general formula 9 is characterized in that the drug is bonded to a bis-iminobiotin compound represented by the general formula 9 by utilizing a functional group J

As the drug, a bioactive substance or a fluorescent compound can be used.

### Effect of invention

According to the present invention, it is possible to provide a bis-iminobiotin compound which has a structure for conveniently binding a bis-iminobiotin moiety and an agent, and which is useful for drug delivery to a substance labeled with streptavidin.

### Detailed description of the present invention

According to one form of the bis-iminobiotin compound according to the present invention, the following reaction is enabled.

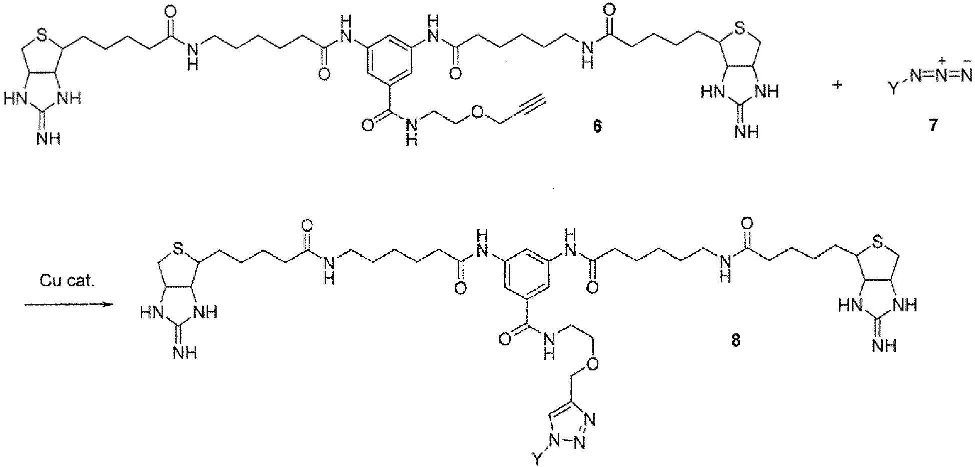

In the above reaction, a terminal having an alkyne structure of a bis-iminobiotin compound represented by formula 6 and a terminal consisting of an azide group of a compound represented by formula 7 are reacted under copper catalyst conditions to obtain a bis-iminobiotin compound represented by formula 8 having a terminal having a triazole ring.

As Y of formula 7 and formula 8, a drug such as a bioactive substance or a fluorescent compound can be used. The compound of formula 8 can be used for pre-targeting and target searching from its molecular structure in the same manner as formula 4 and formula 5 described above.

Because the Huisgen cyclization reaction, which is frequently utilized in the click reaction, occurs functional group selectively, modifications such as protective groups are not required for other parts of formulas 6 and 7 in the above reaction. This makes it possible to combine two molecules at the end of the synthesis. In addition, a compound in which a bis-iminobiotin moiety is co-structured and an alkyne structure and an azide group are replaced can be similarly reacted, thereby enabling flexible synthesis tailored to the compound.

Thus, in order to synthesize a bis-iminobiotin compound useful as an active ingredient of a drug delivery agent of formula 8, a compound such as formula 6 as a substrate for drug delivery is required.

Hereinafter, the present invention will be described in detail.

The bis-iminobiotin compound useful as a substrate for drug delivery is represented by the following general formula 9:

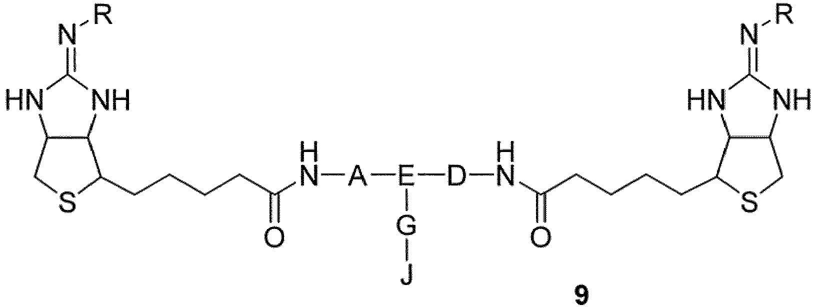

(In the formula, A, D, and E are spacers connecting two bicyclo rings, and E represents a structure capable of taking a branch, and may each have a substituent, and may form a ring structure. J represents a functional group for performing a click reaction, G represents a spacer for bonding E and J, and R represents a hydrogen, an acetyl group, a benzyl group, a trifluoroacetyl group or a Boc group.)

However, the following compounds 10-24 and 10-25 are removed from the above-mentioned bis-iminobiotin compound shown by the general formula 9

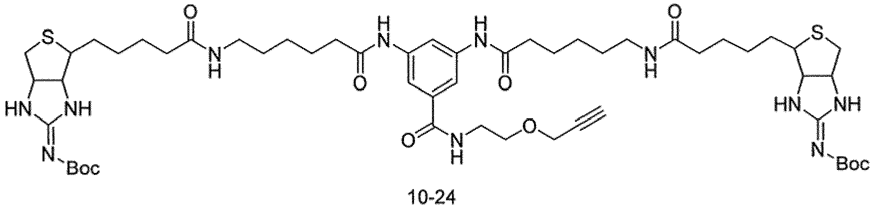

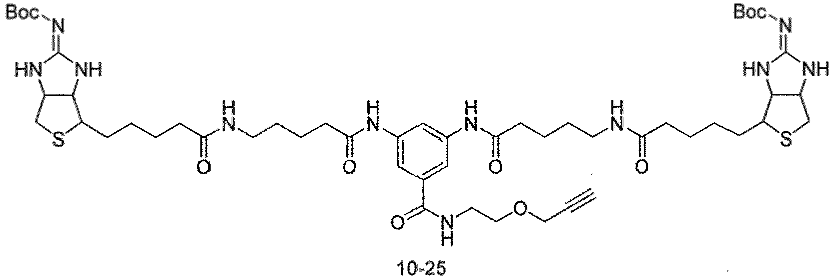

A substrate for drug delivery comprising a bis-iminobiotin compound represented by the above and being utilized for drug delivery to a substance labeled with streptavidin.

As the portion to be bonded to A, D and G of E, nitrogen atoms, carbon atoms, amide groups, benzene rings or heterocycles (e.g., furan rings, pyrimidine rings, pyrrole rings, pyridine rings, etc.) can be preferably used.

The ring structure and the substituent possessed by A, D and E can be selected in the range in which the object effect of the present invention is obtained.

As a portion composed of A, D, and E, a portion in which A is consisting of a1-a2-a3-a4, D is consisting of d1-d2-d3-d4, and A-E-D is represented by a1-a2-a3-a4-E-d1-d2-d3-d4, and a1, a2, a3, a4, E, d1, d2, d3, and d4 are independently selected from the respective columns of Table 1 below is preferable.

**Table 1**

| A | | | | E | D | | | |
|---|---|---|---|---|---|---|---|---|
| a1 | a2 | a3 | a4 | | d4 | d3 | d2 | d1 |
| (CH2)4 | (CH2)1 | CO | NH | Ph | NH | CO | (CH2)1 | (CH2)4 |
| (CH2)3 | (CH2)2 | NH | CO | N | CO | NH | (CH2)2 | (CH2)3 |
| (CH2)2 | NH | NHCO | NHCO | CH | NHCO | NHCO | NH | (CH2)2 |
| (CH2)5 | CO | Bond | (CH2)1 | Hetero ring | (CH2)1 | Bond | CO | (CH2)5 |
| (CH2)6 | O | | (CH2)2 | | (CH2)2 | | O | (CH2)6 |
| (CH2)7 | (CH2)3 | | (CH2)3 | | (CH2)3 | | (CH2)3 | (CH2)7 |
| (CH2)8 | (CH2)4 | | (CH2)4 | | (CH2)4 | | (CH2)4 | (CH2)8 |
| CH(COOH) | (CH2)5 | | (CH2)5 | | (CH2)5 | | (CH2)5 | CH(COOH) |
| CH(COOMe) | CH(COOH) | | Bond | | Bond | | CH(COOH) | CH(COOMe) |
| Bond | CH(COOMe) | | | | | | CH(COOMe) | Bond |
| | Bond | | | | | | Bond | |

"Bond " as described in Tables 1 to 4 in the present invention means a direct bond that directly bonds adjacent groups.

As A-E-D, a structure selected from combinations 1 to 113 of Tables 2 to 4 below is preferred.

**Table 2**

| | A | | | | E | D | | | |
|---|---|---|---|---|---|---|---|---|---|
| | a1 | a2 | a3 | a4 | | d4 | d3 | d2 | d1 |
| 1 | (CH2)4 | CO | NH | Bond | Ph | Bond | NH | CO | (CH2)4 |
| 2 | (CH2)5 | CO | NH | Bond | Ph | Bond | NH | CO | (CH2)5 |
| 3 | (CH2)5 | Bond | Bond | Bond | N | Bond | Bond | Bond | (CH2)5 |
| 4 | (CH2)5 | Bond | Bond | Bond | N | Bond | Bond | Bond | (CH2)6 |
| 5 | (CH2)6 | Bond | Bond | Bond | N | Bond | Bond | Bond | (CH2)6 |
| 6 | (CH2)6 | Bond | Bond | Bond | N | Bond | Bond | Bond | (CH2)7 |
| 7 | (CH2)7 | Bond | Bond | Bond | N | Bond | Bond | Bond | (CH2)7 |
| 8 | (CH2)7 | Bond | Bond | Bond | N | Bond | Bond | Bond | (CH2)8 |
| 9 | (CH2)8 | Bond | Bond | Bond | N | Bond | Bond | Bond | (CH2)8 |
| 10 | (CH2)8 | Bond | Bond | Bond | N | Bond | Bond | Bond | (CH2)9 |
| 11 | (CH2)9 | Bond | Bond | Bond | N | Bond | Bond | Bond | (CH2)9 |
| 12 | (CH2)10 | Bond | Bond | Bond | N | Bond | Bond | Bond | (CH2)10 |
| 13 | (CH2)11 | Bond | Bond | Bond | N | Bond | Bond | Bond | (CH2)11 |
| 14 | (CH2)10 | CO | NH | Bond | Ph | Bond | NH | CO | (CH2)10 |
| 15 | (CH2)9 | CO | NH | Bond | Ph | Bond | NH | CO | (CH2)9 |
| 16 | (CH2)8 | CO | NH | Bond | Ph | Bond | NH | CO | (CH2)8 |
| 17 | (CH2)7 | CO | NH | Bond | Ph | Bond | NH | CO | (CH2)7 |
| 18 | (CH2)6 | CO | NH | Bond | Ph | Bond | NH | CO | (CH2)6 |
| 19 | (CH2)4 | CO | NH | Bond | Ph | Bond | NH | CO | (CH2)5 |
| 20 | (CH2)3 | CO | NH | Bond | Ph | Bond | NH | CO | (CH2)4 |
| 21 | (CH2)3 | CO | NH | Bond | Ph | Bond | NH | CO | (CH2)3 |
| 22 | (CH2)7 | NH | CO | Bond | Ph | Bond | CO | NH | (CH2)7 |
| 23 | (CH2)6 | NH | CO | Bond | Ph | Bond | CO | NH | (CH2)6 |
| 24 | (CH2)5 | NH | CO | Bond | Ph | Bond | CO | NH | (CH2)5 |
| 25 | (CH2)4 | NH | CO | Bond | Ph | Bond | CO | NH | (CH2)5 |
| 26 | (CH2)4 | NH | CO | Bond | Ph | Bond | CO | NH | (CH2)4 |
| 27 | (CH2)3 | NH | CO | Bond | Ph | Bond | CO | NH | (CH2)4 |
| 28 | (CH2)3 | NH | CO | Bond | Ph | Bond | CO | NH | (CH2)3 |
| 29 | CH(COOH) | (CH2)2 | CO | NH | Ph | NH | CO | (CH2)2 | CH(COOH) |
| 30 | CH(COOH) | (CH2)2 | CO | NH | Ph | NH | CO | (CH2)3 | CH(COOH) |
| 31 | CH(COOH) | (CH2)3 | CO | NH | Ph | NH | CO | (CH2)3 | CH(COOH) |
| 32 | CH(COOH) | (CH2)3 | CO | NH | Ph | NH | CO | (CH2)4 | CH(COOH) |
| 33 | CH(COOH) | (CH2)4 | CO | NH | Ph | NH | CO | (CH2)4 | CH(COOH) |
| 34 | CH(COOH) | (CH2)4 | CO | NH | Ph | NH | CO | (CH2)5 | CH(COOH) |
| 35 | (CH2)2 | CH(COOH) | CO | NH | Ph | NH | CO | CH(COOH) | (CH2)2 |
| 36 | (CH2)2 | CH(COOH) | CO | NH | Ph | NH | CO | CH(COOH) | (CH2)3 |
| 37 | (CH2)3 | CH(COOH) | CO | NH | Ph | NH | CO | CH(COOH) | (CH2)3 |
| 38 | (CH2)3 | CH(COOH) | CO | NH | Ph | NH | CO | CH(COOH) | (CH2)4 |
| 39 | (CH2)4 | CH(COOH) | CO | NH | Ph | NH | CO | CH(COOH) | (CH2)4 |
| 40 | (CH2)4 | CH(COOH) | CO | NH | Ph | NH | CO | CH(COOH) | (CH2)5 |
| 41 | (CH2)2 | CH(COOH) | NH | CO | Ph | CO | NH | CH(COOH) | (CH2)2 |
| 42 | (CH2)2 | CH(COOH) | NH | CO | Ph | CO | NH | CH(COOH) | (CH2)3 |
| 43 | (CH2)3 | CH(COOH) | NH | CO | Ph | CO | NH | CH(COOH) | (CH2)3 |
| 44 | (CH2)3 | CH(COOH) | NH | CO | Ph | CO | NH | CH(COOH) | (CH2)4 |
| 45 | (CH2)4 | CH(COOH) | NH | CO | Ph | CO | NH | CH(COOH) | (CH2)4 |
| 46 | (CH2)4 | CH(COOH) | NH | CO | Ph | CO | NH | CH(COOH) | (CH2)5 |
| 47 | CH(COOH) | (CH2)2 | NH | CO | Ph | CO | NH | (CH2)2 | CH(COOH) |
| 48 | CH(COOH) | (CH2)2 | NH | CO | Ph | CO | NH | (CH2)3 | CH(COOH) |
| 49 | CH(COOH) | (CH2)3 | NH | CO | Ph | CO | NH | (CH2)3 | CH(COOH) |
| 50 | CH(COOH) | (CH2)3 | NH | CO | Ph | CO | NH | (CH2)4 | CH(COOH) |

**Table 3**

| | A | | | | E | D | | | |
|---|---|---|---|---|---|---|---|---|---|
| | a1 | a2 | a3 | a4 | | d4 | d3 | d2 | d1 |
| 51 | CH(COOH) | (CH2)4 | NH | CO | Ph | CO | NH | (CH2)4 | CH(COOH) |
| 52 | CH(COOH) | (CH2)4 | NH | CO | Ph | CO | NH | (CH2)5 | CH(COOH) |
| 53 | CH(COOMe) | (CH2)2 | CO | NH | Ph | NH | CO | (CH2)2 | CH(COOMe) |
| 54 | CH(COOMe) | (CH2)2 | CO | NH | Ph | NH | CO | (CH2)3 | CH(COOMe) |
| 55 | CH(COOMe) | (CH2)3 | CO | NH | Ph | NH | CO | (CH2)3 | CH(COOMe) |
| 56 | CH(COOMe) | (CH2)3 | CO | NH | Ph | NH | CO | (CH2)4 | CH(COOMe) |
| 57 | CH(COOMe) | (CH2)4 | CO | NH | Ph | NH | CO | (CH2)4 | CH(COOMe) |
| 58 | CH(COOMe) | (CH2)4 | CO | NH | Ph | NH | CO | (CH2)5 | CH(COOMe) |
| 59 | (CH2)2 | CH(COOMe) | CO | NH | Ph | NH | CO | CH(COOMe) | (CH2)2 |
| 60 | (CH2)2 | CH(COOMe) | CO | NH | Ph | NH | CO | CH(COOMe) | (CH2)3 |
| 61 | (CH2)3 | CH(COOMe) | CO | NH | Ph | NH | CO | CH(COOMe) | (CH2)3 |
| 62 | (CH2)3 | CH(COOMe) | CO | NH | Ph | NH | CO | CH(COOMe) | (CH2)4 |
| 63 | (CH2)4 | CH(COOMe) | CO | NH | Ph | NH | CO | CH(COOMe) | (CH2)4 |
| 64 | (CH2)4 | CH(COOMe) | CO | NH | Ph | NH | CO | CH(COOMe) | (CH2)5 |
| 65 | (CH2)2 | CH(COOMe) | NH | CO | Ph | CO | NH | CH(COOMe) | (CH2)2 |
| 66 | (CH2)2 | CH(COOMe) | NH | CO | Ph | CO | NH | CH(COOMe) | (CH2)3 |
| 67 | (CH2)3 | CH(COOMe) | NH | CO | Ph | CO | NH | CH(COOMe) | (CH2)3 |
| 68 | (CH2)3 | CH(COOMe) | NH | CO | Ph | CO | NH | CH(COOMe) | (CH2)4 |
| 69 | (CH2)4 | CH(COOMe) | NH | CO | Ph | CO | NH | CH(COOMe) | (CH2)4 |
| 70 | (CH2)4 | CH(COOMe) | NH | CO | Ph | CO | NH | CH(COOMe) | (CH2)5 |
| 71 | CH(COOMe) | (CH2)2 | NH | CO | Ph | CO | NH | (CH2)2 | CH(COOMe) |
| 72 | CH(COOMe) | (CH2)2 | NH | CO | Ph | CO | NH | (CH2)3 | CH(COOMe) |
| 73 | CH(COOMe) | (CH2)3 | NH | CO | Ph | CO | NH | (CH2)3 | CH(COOMe) |
| 74 | CH(COOMe) | (CH2)3 | NH | CO | Ph | CO | NH | (CH2)4 | CH(COOMe) |
| 75 | CH(COOMe) | (CH2)4 | NH | CO | Ph | CO | NH | (CH2)4 | CH(COOMe) |
| 76 | CH(COOMe) | (CH2)4 | NH | CO | Ph | CO | NH | (CH2)5 | CH(COOMe) |
| 77 | (CH2)3 | NH | CO | (CH2) | N | (CH2) | CO | NH | (CH2)3 |
| 78 | (CH2)3 | NH | CO | (CH2) | N | (CH2) | CO | NH | (CH2)4 |
| 79 | (CH2)4 | NH | CO | (CH2) | N | (CH2) | CO | NH | (CH2)4 |
| 80 | (CH2)4 | NH | CO | (CH2) | N | (CH2) | CO | NH | (CH2)5 |
| 81 | (CH2)5 | NH | CO | (CH2) | N | (CH2) | CO | NH | (CH2)5 |
| 82 | (CH2)5 | NH | CO | (CH2) | N | (CH2) | CO | NH | (CH2)6 |
| 83 | CH(COOMe) | (CH2)3 | NHCO | (CH2) | N | (CH2) | NHCO | (CH2)3 | CH(COOMe) |
| 84 | CH(COOMe) | (CH2)4 | NHCO | (CH2) | N | (CH2) | NHCO | (CH2)4 | CH(COOMe) |
| 85 | CH(COOMe) | (CH2)5 | NHCO | (CH2) | N | (CH2) | NHCO | (CH2)5 | CH(COOMe) |
| 86 | (CH2)2 | NH | CO | (CH2)2 | N | (CH2)2 | CO | NH | (CH2)2 |
| 87 | (CH2)2 | NH | CO | (CH2)2 | N | (CH2)2 | CO | NH | (CH2)3 |
| 88 | (CH2)3 | NH | CO | (CH2)2 | N | (CH2)2 | CO | NH | (CH2)3 |
| 89 | (CH2)3 | NH | CO | (CH2)2 | N | (CH2)2 | CO | NH | (CH2)4 |
| 90 | (CH2)4 | NH | CO | (CH2)2 | N | (CH2)2 | CO | NH | (CH2)4 |
| 91 | (CH2)4 | NH | CO | (CH2)2 | N | (CH2)2 | CO | NH | (CH2)5 |
| 92 | (CH2)3 | CO | NH | Bond | CH | (CH2) | NH | CO | (CH2)3 |
| 93 | (CH2)3 | CO | NH | Bond | CH | (CH2) | NH | CO | (CH2)4 |
| 94 | (CH2)4 | CO | NH | Bond | CH | (CH2) | NH | CO | (CH2)4 |
| 95 | (CH2)4 | CO | NH | Bond | CH | (CH2) | NH | CO | (CH2)5 |
| 96 | (CH2)5 | CO | NH | Bond | CH | (CH2) | NH | CO | (CH2)5 |
| 97 | (CH2)5 | CO | NH | Bond | CH | (CH2) | NH | CO | (CH2)6 |
| 98 | (CH2)6 | CO | NH | Bond | CH | (CH2) | NH | CO | (CH2)6 |
| 99 | (CH2)3 | CO | NH | (CH2) | CH | (CH2) | NH | CO | (CH2)3 |
| 100 | (CH2)3 | CO | NH | (CH2) | CH | (CH2) | NH | CO | (CH2)4 |

**Table 4**

| | A | | | | E | D | | | |
|---|---|---|---|---|---|---|---|---|---|
| | a1 | a2 | a3 | a4 | | d4 | d3 | d2 | d1 |
| 101 | (CH2)4 | CO | NH | (CH2) | CH | (CH2) | NH | CO | (CH2)4 |
| 102 | (CH2)4 | CO | NH | (CH2) | CH | (CH2) | NH | CO | (CH2)5 |
| 103 | (CH2)5 | CO | NH | (CH2) | CH | (CH2) | NH | CO | (CH2)5 |
| 104 | Bond | (CH2)3 | NH | CO | CH | CO | NH | (CH2)3 | Bond |
| 105 | Bond | (CH2)3 | NH | CO | CH | CO | NH | (CH2)4 | Bond |
| 106 | Bond | (CH2)4 | NH | CO | CH | CO | NH | (CH2)4 | Bond |
| 107 | Bond | (CH2)4 | NH | CO | CH | CO | NH | (CH2)5 | Bond |
| 108 | Bond | (CH2)5 | NH | CO | CH | CO | NH | (CH2)5 | Bond |
| 109 | Bond | (CH2)5 | NH | CO | CH | CO | NH | (CH2)6 | Bond |
| 110 | Bond | (CH2)6 | NH | CO | CH | CO | NH | (CH2)6 | Bond |
| 111 | CH(COOH) | (CH2)3 | NHCO | (CH2) | N | (CH2) | NHCO | (CH2)3 | CH(COOH) |
| 112 | CH(COOH) | (CH2)4 | NHCO | (CH2) | N | (CH2) | NHCO | (CH2)4 | CH(COOH) |
| 113 | CH(COOH) | (CH2)5 | NHCO | (CH2) | N | (CH2) | NHCO | (CH2)5 | CH(COOH) |

As a more preferable bis-iminobiotin compound according to the present invention, a compound represented by the following general formulas (10-1) to (10-38) can be mentioned.

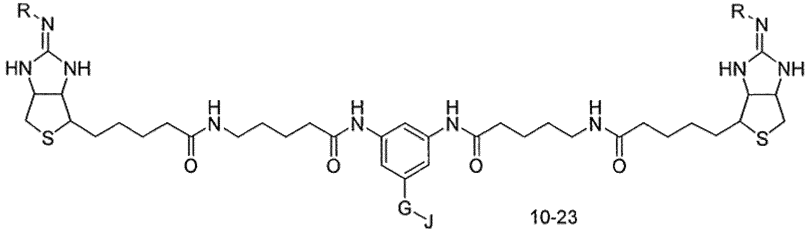

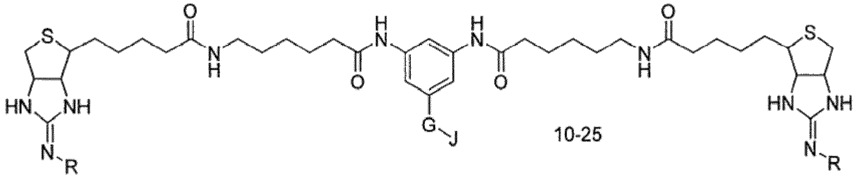

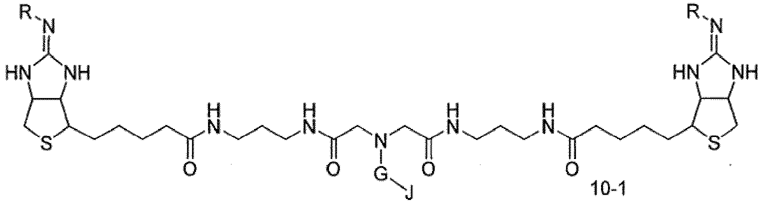

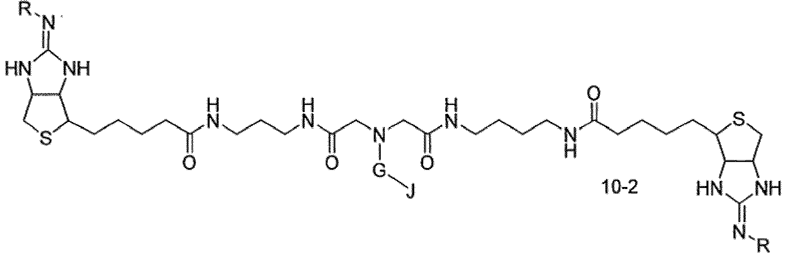

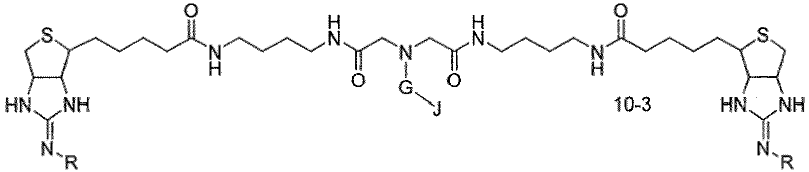

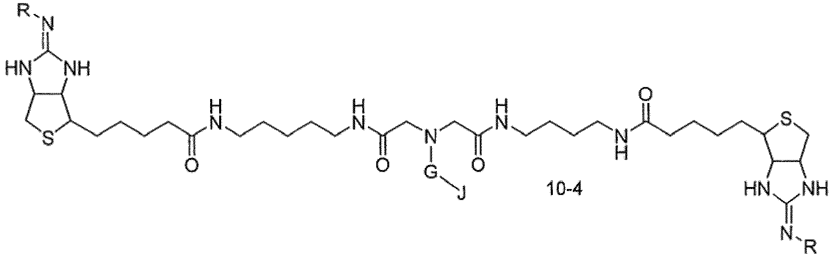

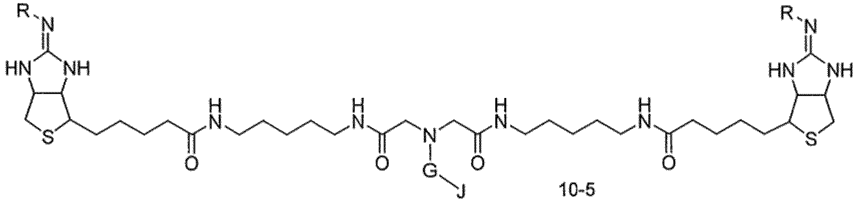

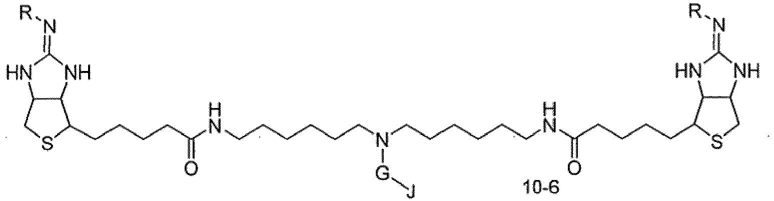

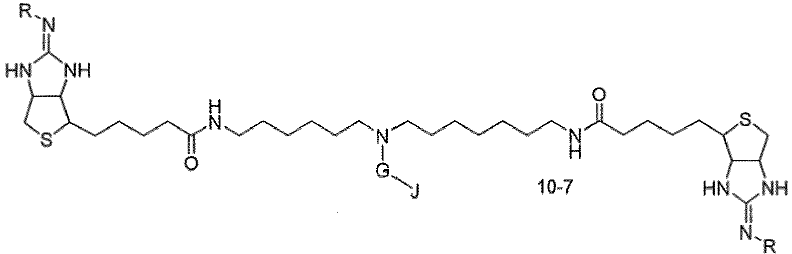

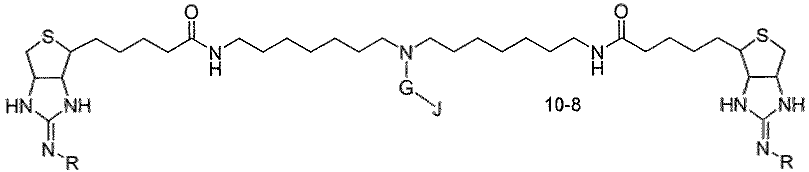

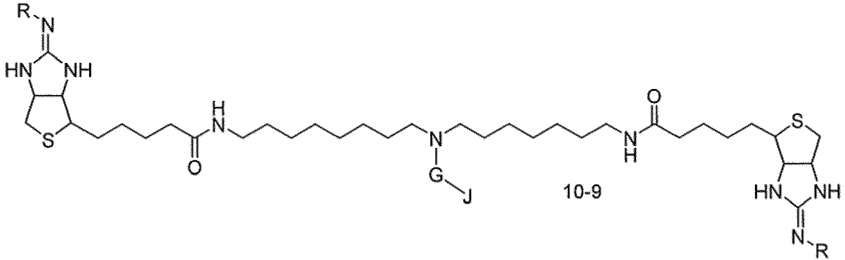

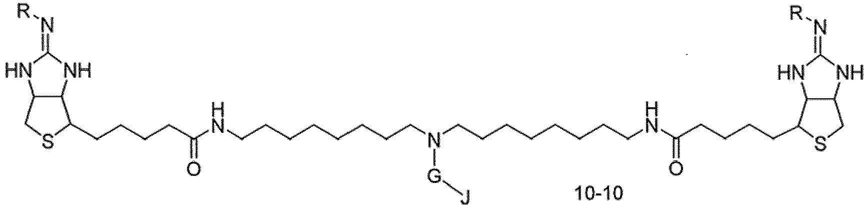

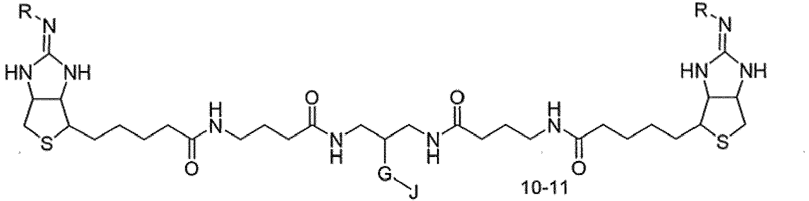

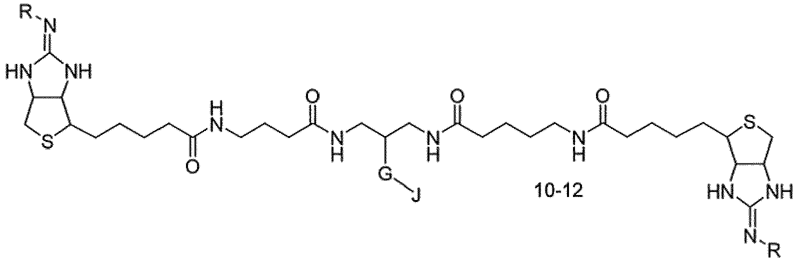

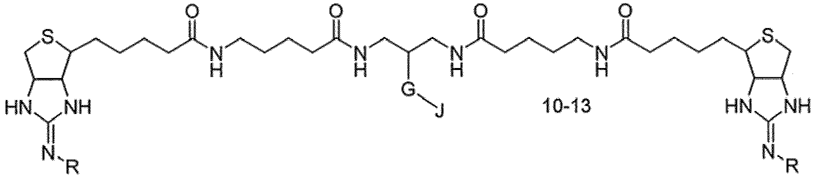

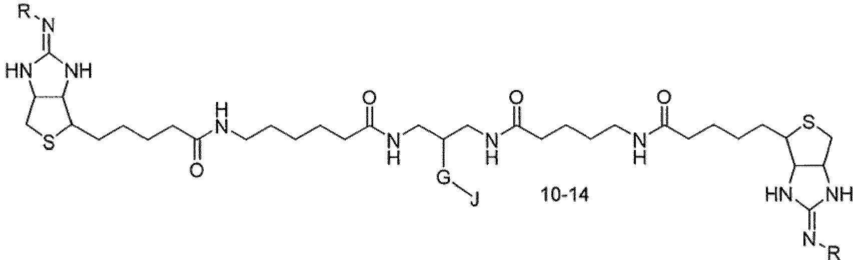

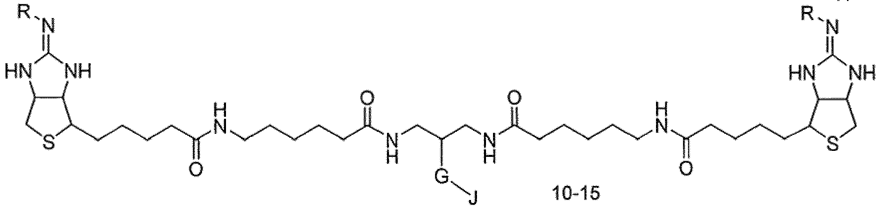

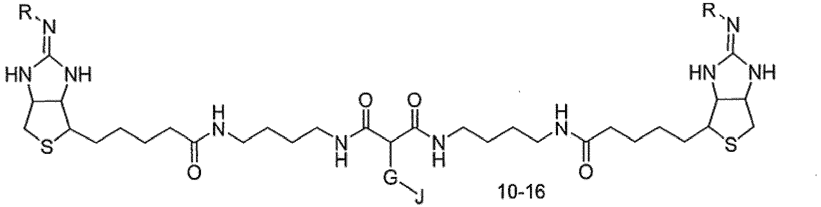

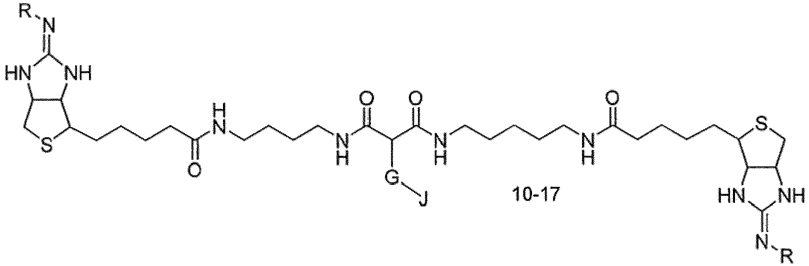

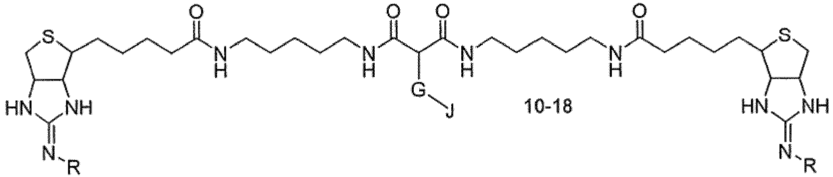

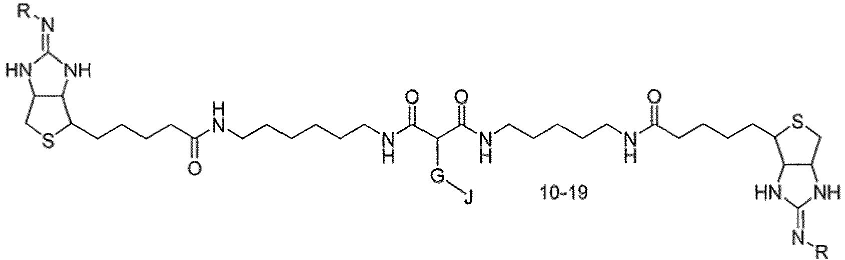

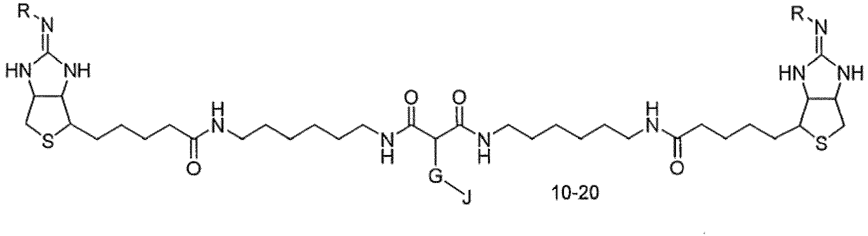

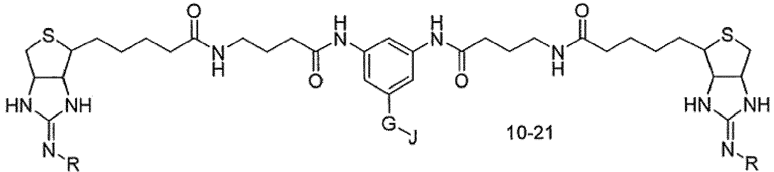

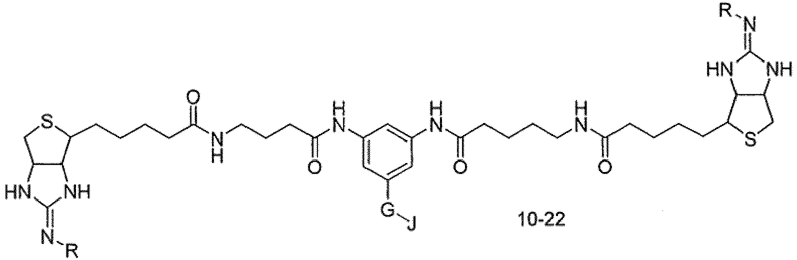

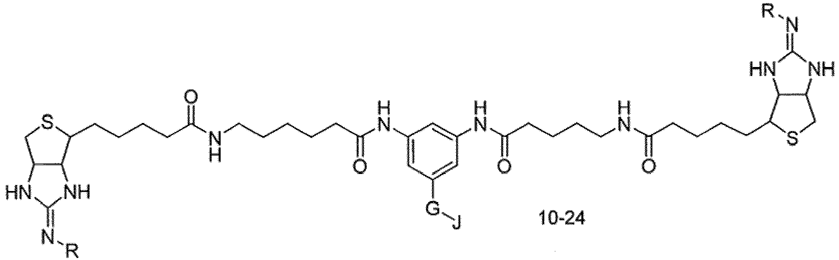

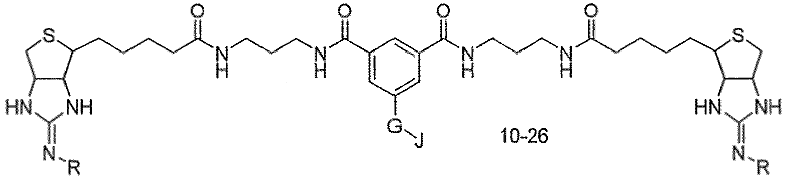

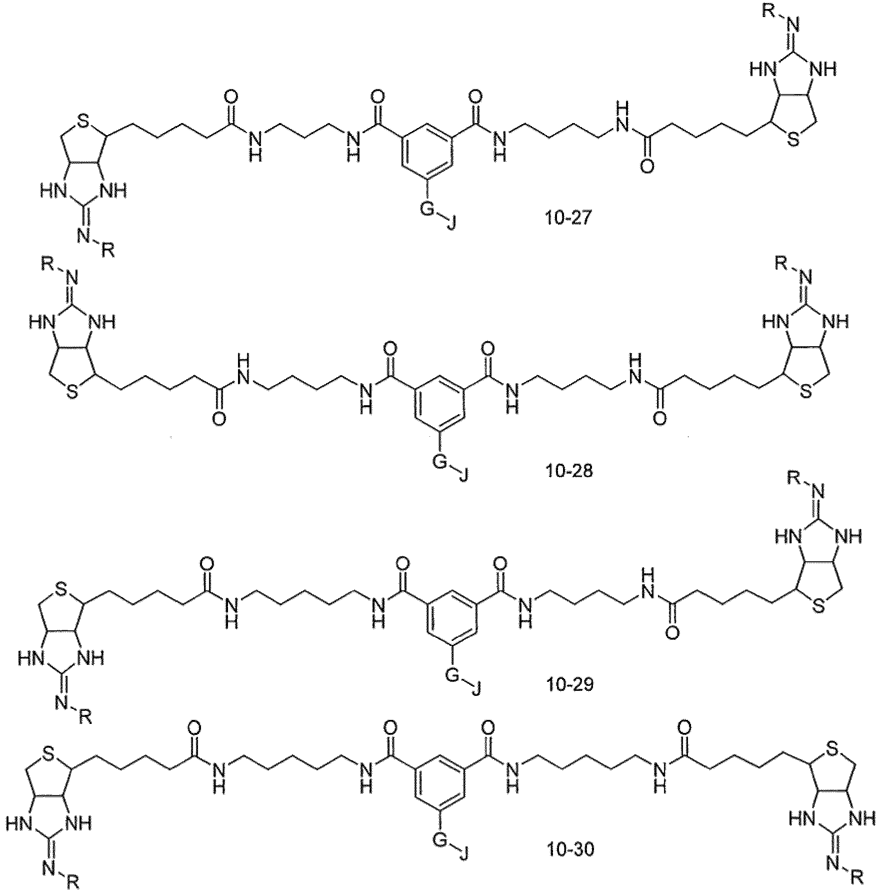

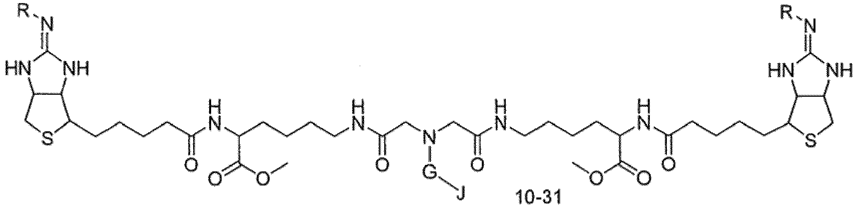

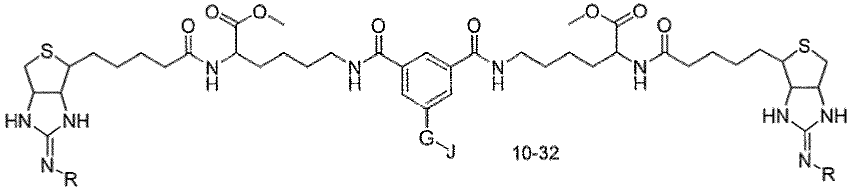

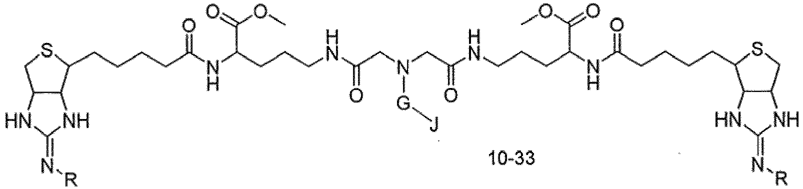

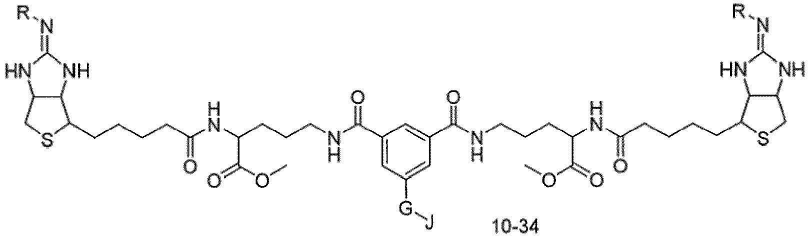

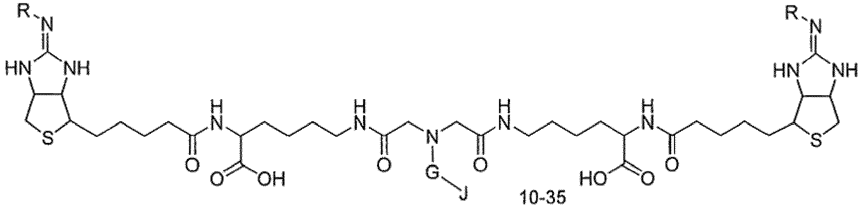

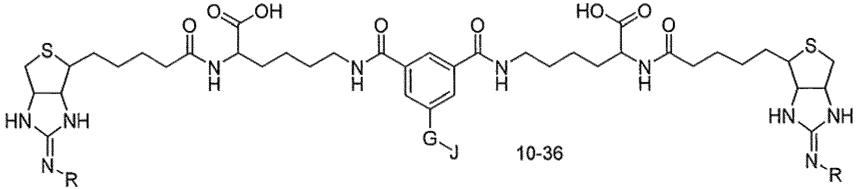

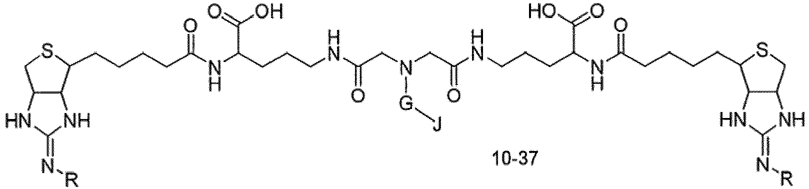

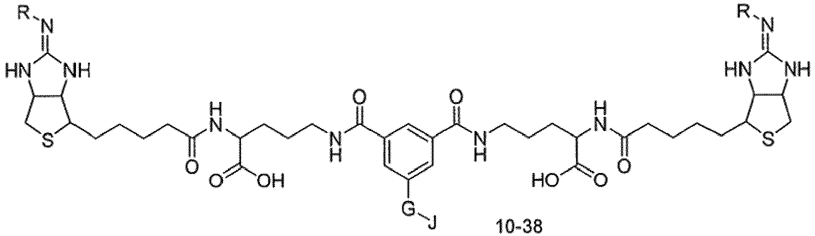

G is a linking group connecting E and J, and is represented by g1-g2-g3-g4-g5-g6-g7, and each of the g1,g2,g3,g4,g5,g6 and g7 is independently preferably a linking group selected from the respective columns of Table 4 below.

**Table 5**

| g1 | g2 | g3 | g4 | g5 | g6 | g7 |
|---|---|---|---|---|---|---|
| NHCO | (CH2)2 | Bond | Bond | Bond | Bond | Bond |
| CO | CO | (OCH2CH2)3 | CO | NH | (CH2)1 | NH |
| O | Ph | O | NHCO | O | (CH2)2 | O |
| NH | (CH2)1 | Ph | (CH2)1 | CO | (CH2)3 | CO |
| (CH2)1 | (CH2)3 | Hetero ring | (CH2)2 | Ph | (CH2)4 | |
| (CH2)2 | (CH2)4 | (CH2)1 | (CH2)3 | Hetero ring | (CH2)5 | |
| (CH2)3 | (CH2)5 | (CH2)2 | (CH2)4 | (OCH2CH2)1 | (CH2)6 | |
| (CH2)4 | (CH2)6 | (CH2)3 | (CH2)5 | (OCH2CH2)2 | | |
| (CH2)5 | (OCH2CH2)1 | (CH2)4 | (CH2)6 | (OCH2CH2)3 | | |
| (CH2)6 | (OCH2CH2)2 | (CH2)5 | (OCH2CH2)1 | (OCH2CH2)4 | | |
| Ph | (OCH2CH2)3 | (CH2)6 | (OCH2CH2)2 | (OCH2CH2)5 | | |
| Hetero ring | (OCH2CH2)4 | (OCH2CH2)1 | (OCH2CH2)3 | (OCH2CH2)6 | | |
| Bond | (OCH2CH2)5 | (OCH2CH2)2 | (OCH2CH2)4 | (OCH2CH2)7 | | |
| | (OCH2CH2)6 | (OCH2CH2)4 | (OCH2CH2)5 | (OCH2CH2)8 | | |
| | (OCH2CH2)7 | (OCH2CH2)5 | (OCH2CH2)6 | (OCH2CH2)9 | | |
| | (OCH2CH2)8 | (OCH2CH2)6 | (OCH2CH2)7 | | | |
| | (OCH2CH2)9 | (OCH2CH2)7 | (OCH2CH2)8 | | | |
| | Bond | (OCH2CH2)8 | (OCH2CH2)9 | | | |
| | | (OCH2CH2)9 | | | | |

Hetero rings in Table 5 may include, for example, furan rings, pyrimidine rings, pyrrole rings, pyridine rings, and the like.

As a preferable linking group as G, a linking group represented by the following general formulas (3-1) to (3-22) can be mentioned.
In each of the following general formulas, J and E are shown to indicate the bonding position with J and E, but G is a portion excluding J and E.

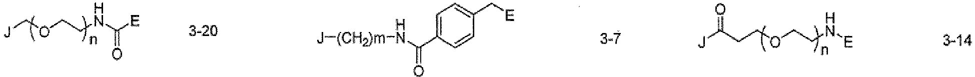

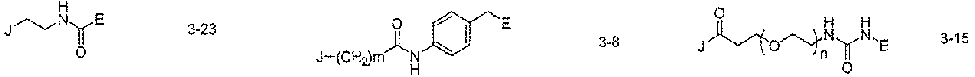

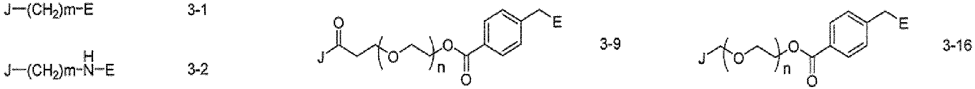

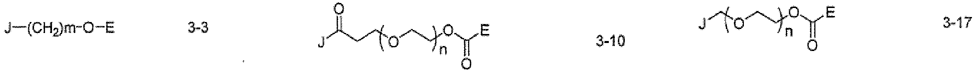

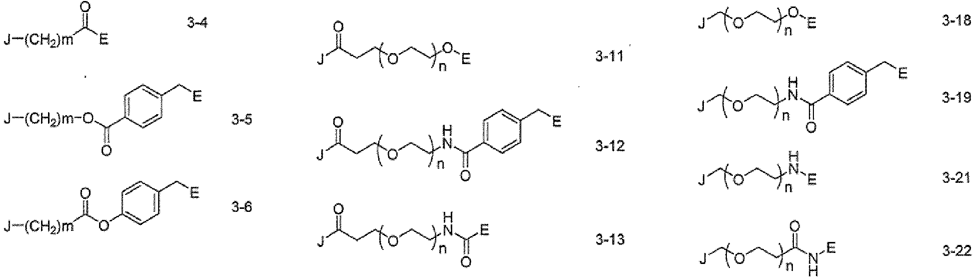

In the above formulae 3-1 to 3-22, m in -(CH2)m- independently represents an integer of 1 to 6, and n in -(OCH2CH2)n- independently represents an integer of 1 to 9.

J is a functional group for performing a click reaction. Such functional groups may include an azide group, a group having an alkyne structure, a tetrazine group, a trans-cyclooctyne group, and the like. Among these, a group having an azide group and an alkyne structure is preferred, and can be selected and used depending on the structure of the binding site of G with J

As a group having an alkyne structure, an alkynyl group, an alkynyloxy group, an alkynylamino group, or the like can be mentioned, and as an alkynyl group contained in these groups, an alkynyl group having 2 to 3 carbon atoms such as an ethynyl group, a propagyl group, or the like can be mentioned.

Examples of the group having a preferred azide group and an alkyne structure include the following groups represented by the following formulas (4-1) to (4-10) In the following formulas, the position at which G is displayed indicates the binding position with G.

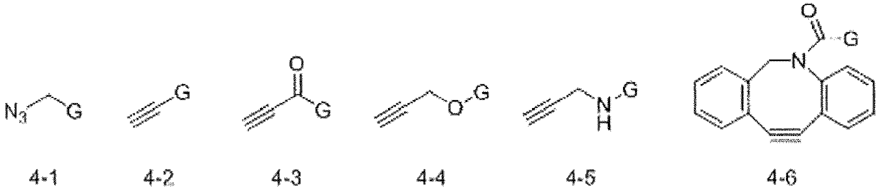

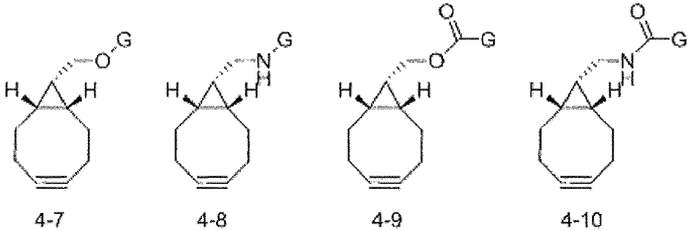

The compounds can be selected in the range where the structures and substituents of R, A, D, E, G, and J are the effects of interest of the present invention.

As R, a hydrogen atom can be preferably used.

As A and D, one structure selected from the following six structures shown in Table 6 having an alkyl chain or an alkyl chain having a substituent is further preferred.

**Table 6**

| | A, D | |
|---|---|---|
| Iminobiotin side | -(CH2)4- | E side |
| | -(CH2)5- | |
| | -(CH2)6- | |
| | -(CH2)7- | |
| | -CH(COOH)CH2CH2CH2CH2- | |
| | -CH(COOH)CH2CH2CH2- | |

As E, one structure selected from the following three structures, having diaminobenzoic acid, monoaminodibenzoic acid or benzylamine as a central skeleton, is further preferred (provided that (A) represents a bond to A, (D) represents a bond to D, and (G) represents a bond to G)

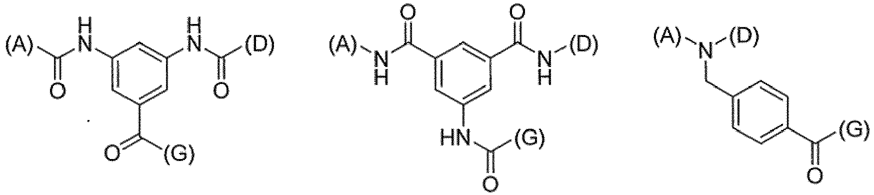

As G, one structure selected from the six structures shown in Table 7 below having ethylene glycol is further preferred.

**Table 7**

| | G | |
|---|---|---|
| E side | -NHCH2CH2OCH2- | J side |
| | -NH(CH2CH2O)3CH2- | |
| | -NH(CH2CH2O)3CH2CH2CONHCH2- | |
| | -NH(CH2CH2O)3CH2CH2COOCH2- | |
| | -NHCH2CH2CO- | |
| | -CH2O(CH2CH2O)2CH2CH2- | |

As J, one structure selected from the following three structures having an alkynyl group or an azide group is further preferred (provided that (G) represents a bond to G)

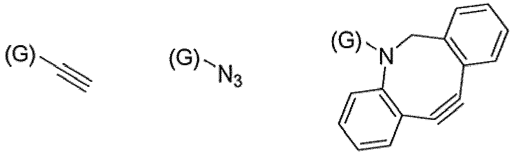

Further preferred compounds as the bis-iminobiotin compounds according to the present invention are shown in the following compounds 13-1 to 13-23

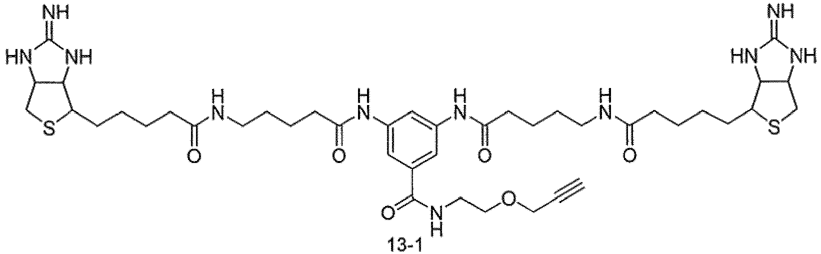

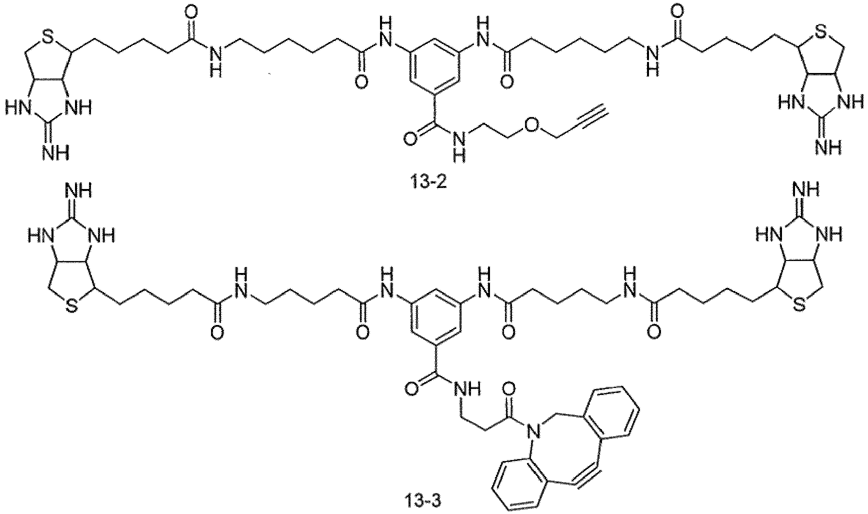

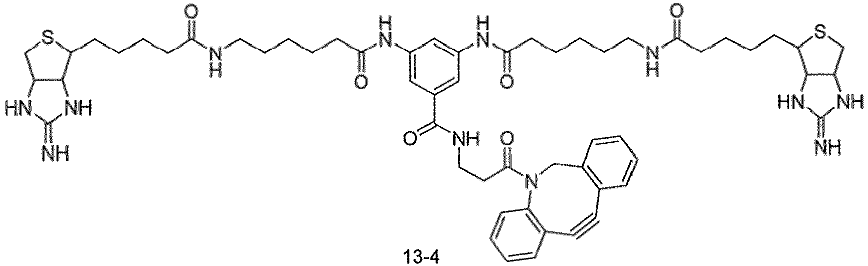

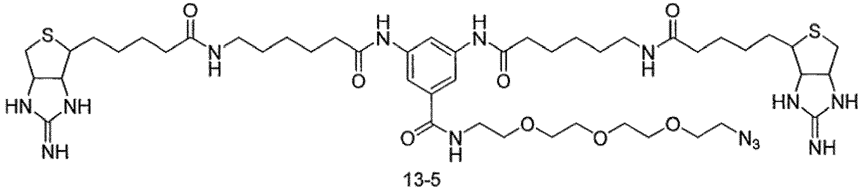

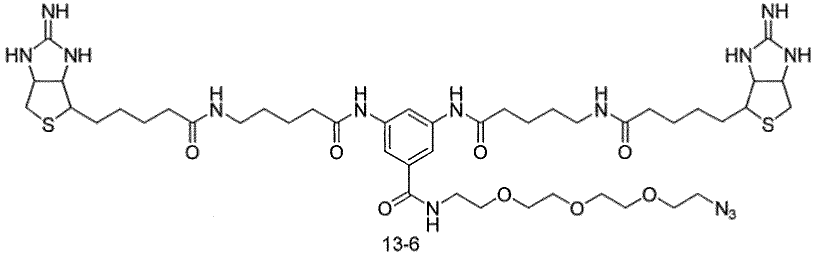

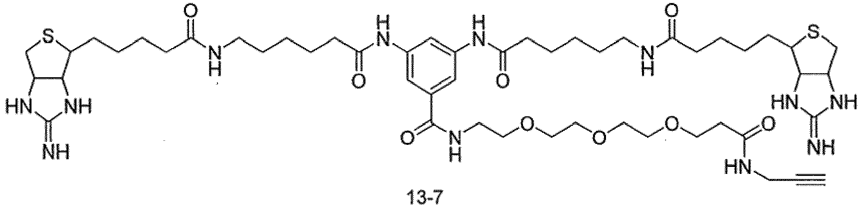

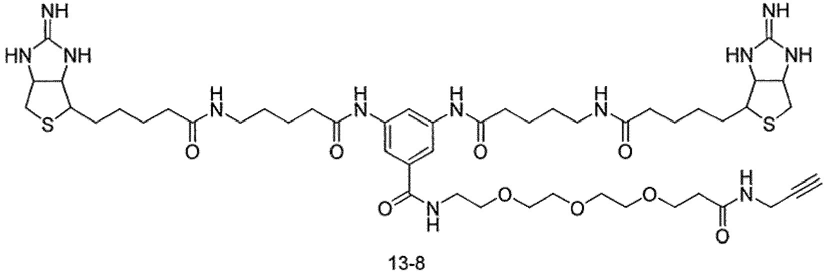

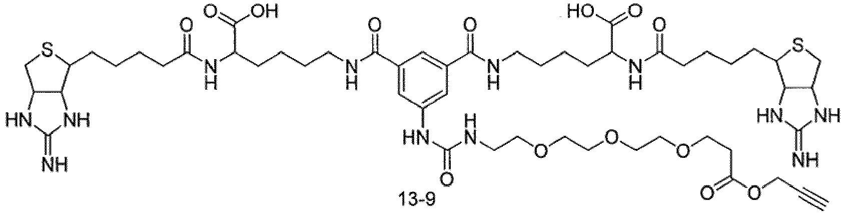

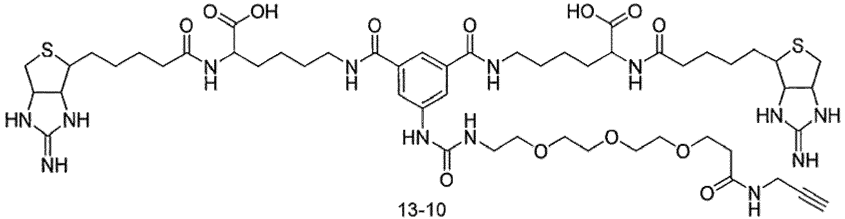

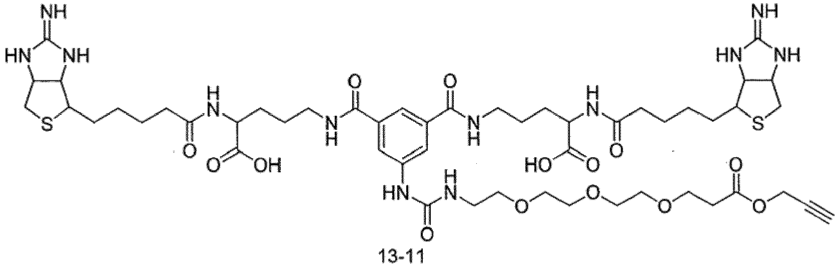

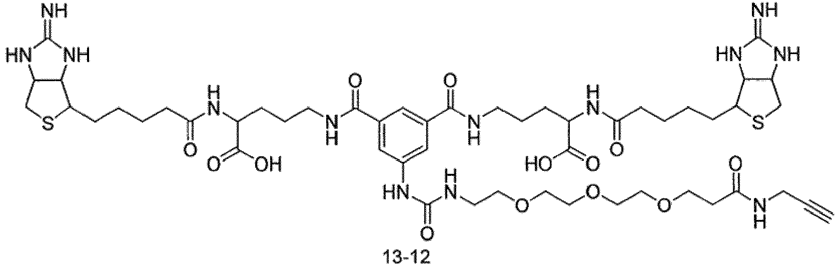

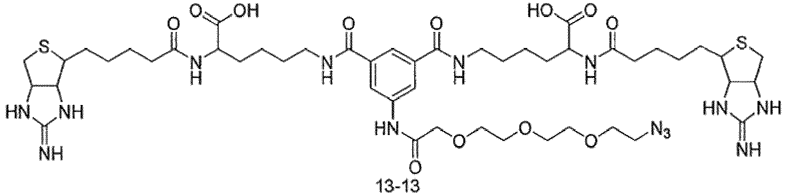

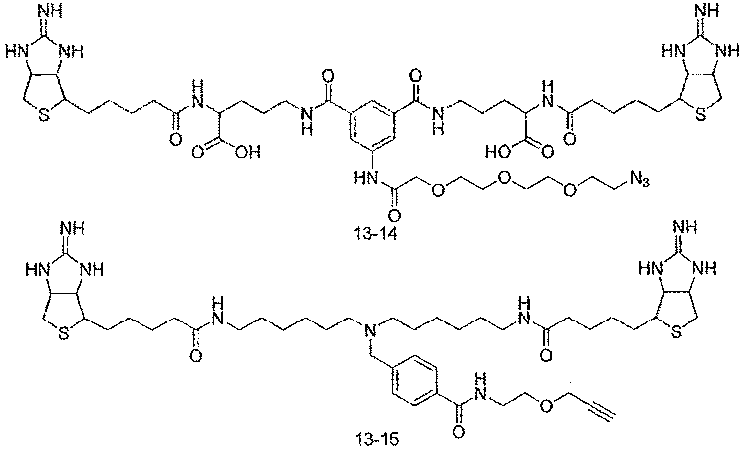

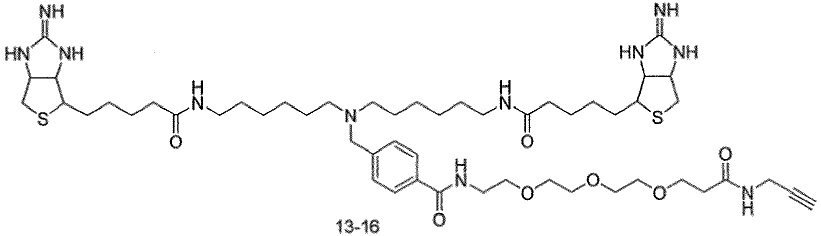

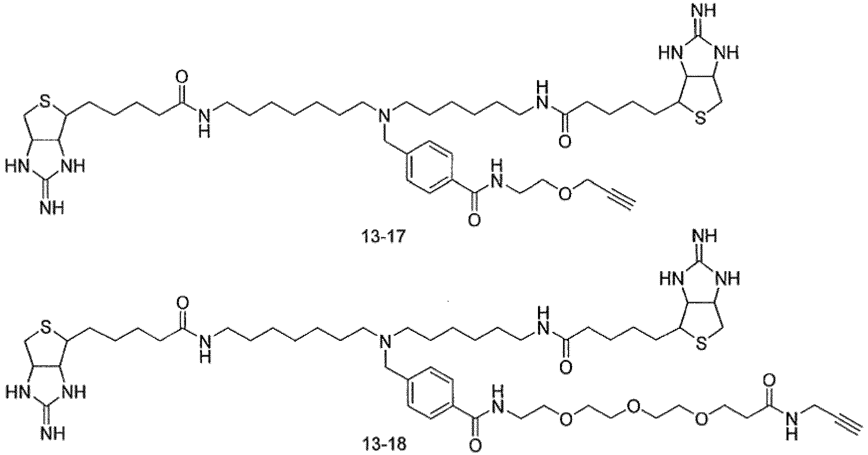

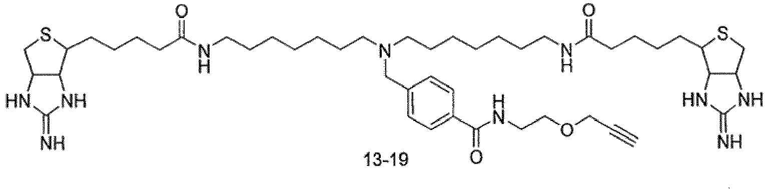

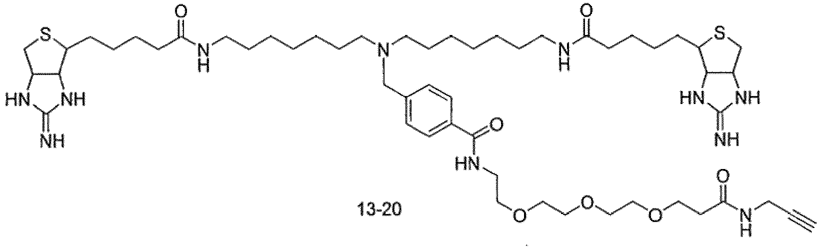

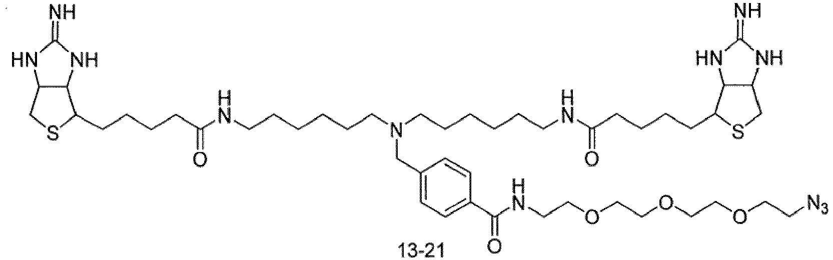

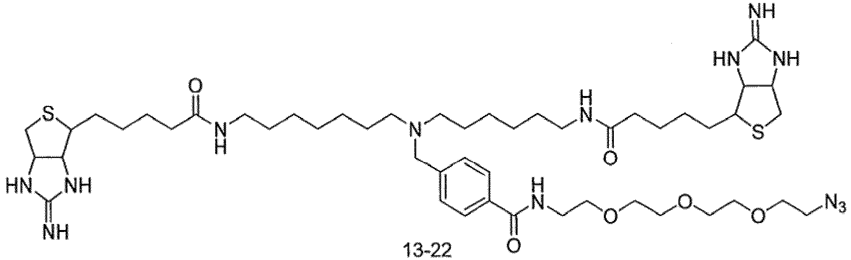

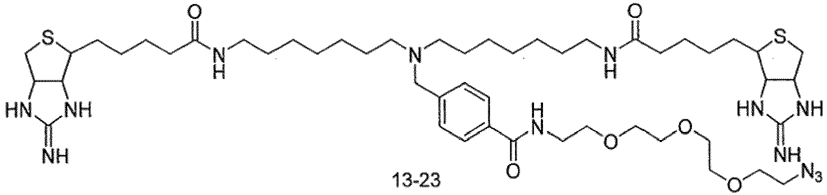

It is particularly preferred that E as a spacer structure is the following structure (provided that (A) represents binding to A, (D) represents binding to D, and (G) represents binding to G)

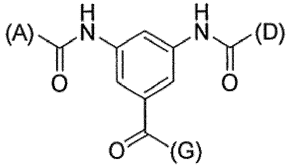

Further preferred compounds having E consisting of the above structure as the spacer structure may include compounds 13-1, 13-2, 13-4 and 13-6 described above.

The drug delivery agent according to the present invention includes, as an active ingredient, a drug-conjugated bis-iminobiotin represented by the following general formula 14 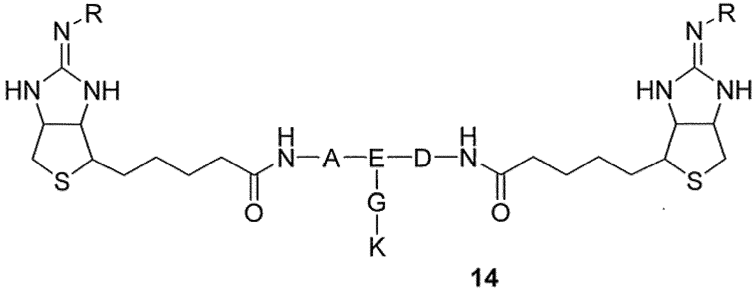 (wherein A, D, E, G, R are defined as in general formula 9, and K represents a drug)

The drug used for derivatization is not particularly limited as long as it has a structure capable of binding to a compound of general formula 9 utilizing a functional group J of general formula 9 and can be used for pre-targeting. Examples of the drug include a bioactive substance or a fluorescent compound.

Specific examples of biologically active substances include anticancer drugs, central nervous system drugs, immune disease drugs, and cardiovascular drugs.

Specific examples of the fluorescent compound include coumarin analogs and cyanine analogs, rhodamine analogs, and fluorescein analogs.

In addition to the compound represented by Formula 9, the drug delivery agent may include a carrier for a formulation, an excipient, a diluent such as a solvent, and the like. Drug delivery agents allow delivery of the drug to a substance or site labeled with streptavidin in vivo, such as an animal, including a human, or in vitro.

Hereinafter, a method for producing a compound represented by general formula 9 will be described.

An ester bond or an amide bond in the structure of general formula 9 can be formed by the following reaction.

An amide of general formula 17 can be formed by condensation of an amine represented by general formula 15 with a carboxylic acid represented by general formula 16

In addition, an amide bond or an ester bond of E and G in the structure of general formula 9 can be formed by condensation of general formulas 17 and 18

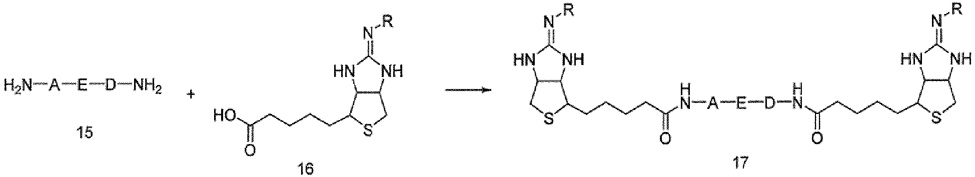

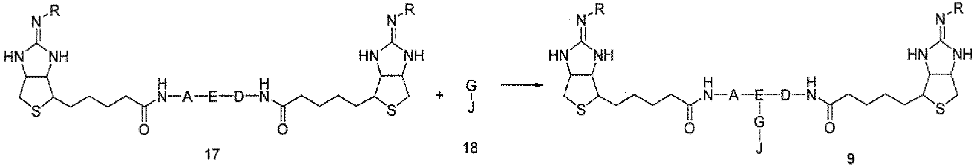

Conditions for condensation reactions that can be utilized in the preparation of general formula 9 are described below.

As the solvent used in the condensation reaction, any one or two or more of N,N-dimethylformamide, acetonitrile, tetrahydrofuran, tetrahydropyran, dichloromethane, 1,4-dioxane, chloroform, toluene, and benzene can be used in combination.

Preferably, any one of N,N-dimethylformamide, acetonitrile, tetrahydrofuran, dichloromethane, and 1,4-dioxane or two or more of these is used in combination as a solvent.

More preferably, N,N-dimethylformamide, acetonitrile, tetrahydrofuran, dichloromethane or 1,4-dioxane is used as a single solvent.

More preferably, N,N-dimethylformamide, acetonitrile or tetrahydrofuran is used as a single solvent.

As a condensing agent of the condensation reaction, a carbodiimide-based condensing agent (e.g., N,N'-dicyclohexylcarbodiimide, N,N'-diisopropylcarbodiimide, 1-ethyl-3-(3-dimethylaminopropyl)carbodiimide hydrochloride, etc.), a benzotriazole-based condensing agent (e.g., O-(7-azabenzotriazol-1-yl)-N,N',N'-tetramethyluronium hexafluorophosphate, O-(benzotriazole-1-yl)-N,N',N'-tetramethyluronium hexafluorophosphate, and the like), a triazole-based condensing agent (e.g., 4-(4,6-dimethoxy-1,3,5-triazin-2-yl)-4-methylmorpholinium chloride, and the like), and a uronium-type condensing agent (e.g., any one of (1-cyano-2-ethoxy-2-oxoethylideneaminooxy) dimethylamino-morpholino-carbeniumhexafluorophosphate and the like) or two or more of these may be used in combination.

Preferably, any one of a carbodiimide-based condensing agent, a benzotriazole-based condensing agent, a triazole-based condensing agent, and a uronium-type condensing agent is used as the condensing agent.

More preferably, any one of a carbodiimide-based condensing agent, a benzotriazole-based condensing agent, and a uronium-type condensing agent is used as the condensing agent.

More preferably, any one of O-(7-azabenzotriazol-1-yl)-N,N',N '-tetramethyluronium hexafluorophosphate, O-(benzotriazol-1-yl)-N,N,N',N'-tetramethyluronium hexafluorophosphate, (1-cyano-2-ethoxy-2-oxoethylidenaminooxy)dimethylamino-morpholino-carbenium hexafluorophosphate is used as the condensing agent.

At least one of acid halides (e.g., acid chloride, acid fluoride, acid bromide, etc.) and active esters (e.g., N-hydroxysuccinimide ester, N-hydroxysulfosuccinimide ester, 1-acyloxy-7-azabenzotriazole, 1-acyloxybenzotriazole, etc.) can be used to activate the carboxylic acid in the condensation reaction.

Preferably, at least one of an acid halide, a succinic ester, and an active ester is used for activating the carboxylic acid.

More preferably, at least one of an acid chloride, a N-hydroxysuccinimide ester, and a N-hydroxysulfosuccinimide ester is used for activation of a carboxylic acid.

More preferably, at least one of an acid chloride and a N-hydroxysuccinimide ester is used for activating the carboxylic acid.

As an additive for the condensation reaction, any one of a tertiary amine (e.g., triethylamine, diisopropylethylamine, trimethylamine, and the like), a 4-dimethylaminopyridine, and an imidazole, or two or more of these may be used in combination.

Preferably, any one of triethylamine, diisopropylethylamine, trimethylamine, and 4-dimethylaminopyridine or 2 or more of these is used in combination as an additive. More preferably, any one of triethylamine, diisopropylethylamine, trimethylamine, and 4-dimethylaminopyridine is used as the additive.

Further preferably, triethylamine or diisopropylethylamine is used as the additive.

The reaction temperature of the condensation reaction may be selected from a range of -78 °C. or more and 150 °C. or less, preferably 0 °C. or more and 150 °C. or less, and more preferably 0 °C. or more and 50 or less.

The reaction substrate concentration of the condensation reaction may be selected from the range of 0.001 mol/L to no solvent, preferably from 0.01 to 10 mol/L

The reaction time of the condensation reaction may be selected from the range of 1 minutes to 100 hours, and more preferably from 30 minutes to 24 hours.

For purification of the condensation reaction, crystallization, filtration washing, silica gel column chromatography, gel filtration chromatography, and the like can be used.

As a preferred purification method, at least one of crystallization, filtration washing and silica gel column chromatography is used.

Hereinafter, a method for producing a compound for delivering a drug represented by general formula 14 will be described.
The bond between G and K of general formula 14 can be formed by the following reaction.

For example, when J in the structure of general formula 9' is an alkyne, a structure represented by general formula 18 is formed by reacting an azide represented by general formula 7 Alternatively, when J in the structure of general formula 9" is an azide, a structure represented by general formula 20 is formed by reacting an azide represented by general formula 19 (However, Y and Z represent structures of bioactive substances or fluorescent compounds that bind with alkynes or azides)

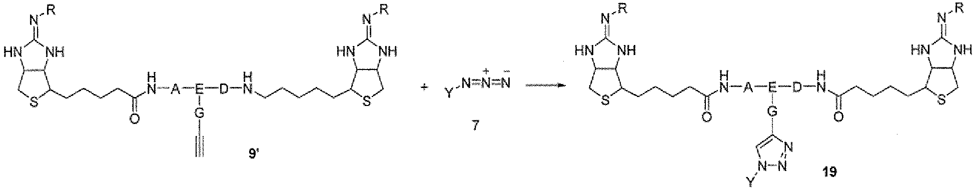

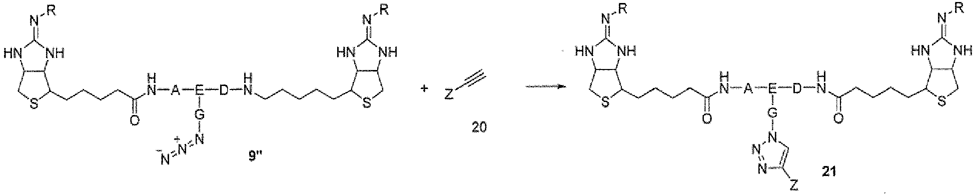

Conditions for cyclization reactions that can be utilized in the preparation of formula 14 are described below.

As a solvent used in the cyclization reaction, any one or two or more of N,N-dimethylformamide, acetonitrile, tetrahydrofuran, tetrahydropyran, dichloromethane, 1,4-dioxane, dimethyl sulfoxide, chloroform, toluene, benzene, water, acetic acid, methanol, ethanol, isopropanol, normal-butyl alcohol, and tartarry-butyl alcohol can be used in combination.

Preferably, any one or two or more of N,N-dimethylformamide, acetonitrile, tetrahydrofuran, 1,4-dioxane, chloroform, toluene, benzene, water, acetic acid, methanol, ethanol, isopropanol, normal-butyl alcohol, and tartarry-butyl alcohol is used in combination as a solvent.

More preferably, any one or two or more of N,N-dimethylformamide, acetonitrile, 1,4-dioxane, water, acetic acid, methanol, ethanol, and tertiary-butyl alcohol is used in combination as a solvent.

More preferably, any one of N,N-dimethylformamide, acetonitrile, 1,4-dioxane, water, acetic acid, ethanol, and tertiary-butyl alcohol is used as the solvent.

As a catalyst used in a cyclization reaction, they are monovalent copper salts (for example, copper(I) chloride, copper(I) bromide, copper(I) iodide, copper(I) acetate, etc.), Any one or 2 or more of divalent copper salts (e.g., copper (II) chloride, copper (II) bromide, copper (II) iodide, copper (II) acetate, copper (II) sulfate, etc.), silver salts (silver chloride, silver bromide, silver iodide, silver acetate, etc.) and ruthenium salts may be used in combination.

Alternatively, the cyclization reaction may be carried out without adding a catalyst.

Preferably, any one of a monovalent copper salt, a divalent copper salt, and a silver salt or two or more of these is used in combination as a catalyst.

More preferably, any one of copper (I) chloride, copper (I) bromide, copper (I) iodide, copper (I) acetate, copper (II) chloride, copper (II) bromide, copper (II) iodide, copper (II) acetate, and copper (II) sulfate is used as the catalyst.

As an additive of the cyclization reaction, any one of ascorbic acid, sodium ascorbate, potassium ascorbate, and calcium ascorbate, or a combination of two or more of these can be used.

Alternatively, the cyclization reaction may be carried out without adding an additive.

As the additive, preferably, any one of ascorbic acid, sodium ascorbate, and potassium ascorbate is used.

Further preferably, sodium ascorbate is used as an additive.

The reaction temperature of the cyclization reaction may be selected from a range of -78 ° C. or more and 150 ° C., preferably a range of 0 ° C. or more and 100 ° C. or less, and more preferably a range of 0 ° C. or more and 60 ° C. or less.

The reaction substrate concentration of the cyclization reaction may be selected from the range of 0.001 mol/L to solvent-free, preferably from 0.01 to 10 mol/L

The reaction time of the cyclization reaction may be selected from the range of 1 minutes to 168 hours, preferably from 30 minutes to 24 hours.

For purification of the cyclization reaction, crystallization, filtration washing, silica gel column chromatography, and gel filtration chromatography can be used.

As a preferred purification method, at least one of crystallization, filtration washing, and silica gel column chromatography is used.

### Examples:

Hereinafter, examples of the present invention will be described, but the present invention is not limited thereto. NMR-analysis values were measured using the Japanese Electronic EX-270 (270 MHz).

HPLC analysis was performed under the following two conditions.

### (Analysis Condition A)

- Columns: YMC-Pack ODS-AM 150 *6mm
- Flow rate: 1 mL/min.
- Column temperature:40 °C
- Detection wavelength: 254 nm
- Mobile phase: 0.1% aqueous trifluoroacetic acid/acetonitrile

### (Analysis Condition B)

- Columns: YMC Triart C18 75 *2mm
- Flow rate: 0.3 mL/min.
- Column temperature: 35°C
- Detection wavelength: 254 nm
- Mobile phase: 0.1% aqueous trifluoroacetic acid/acetonitrile

Gradient conditions are described as, for example, 0.1% aqueous trifluoroacetic acid/acetonitrile = 85/15 (12 min) 35/65, but represent conditions in which 0.1% aqueous trifluoroacetic acid is reduced from 85% to 35% over 12 min and subsequently returned to 85%

The examples are described below as synthetic flows.

### EXAMPLE 1-1

### (Synthesis of Bis(Boc-iminobiotin) Compound 24)

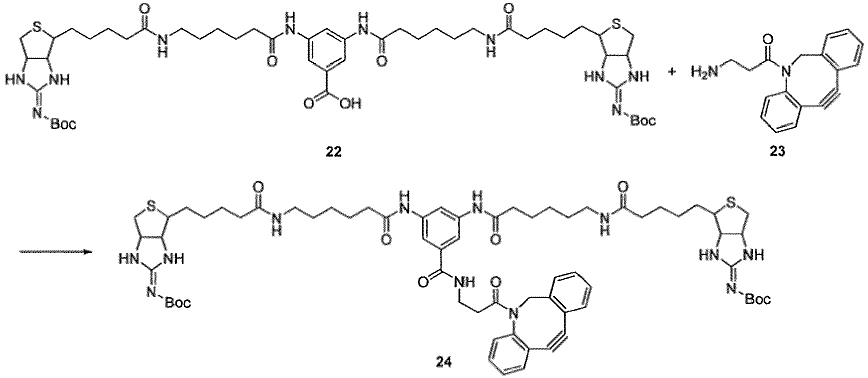

To 135 mg (0.13 mmol) of bis (Boc-iminobiotin) 22 was added 1 mL of dehydrated dimethylformamide and an additional 22 mg (1.05 equiv) of carbonyldiimidazole. 40 °C for 1 h, and 36 mg (1 equiv) of amine 23 (Shigma-Aldrich, CAS No; 1255942-06-3) dissolved in 0.7 mL of dehydrated chloroform was added and stirred at room temperature for 5 h. After completion of the reaction, the mixture was concentrated under reduced pressure, 10 mL of chloroform was added, and the mixture was adjusted to pH 5 with 0.1 N hydrochloric acid. A precipitate occurred and water and chloroform were removed. The precipitate was dissolved in methanol, dried over magnesium sulfate and concentrated to give a residue. Was purified on a silica gel column (chloroform/methanol = 20/1 to 5/1) to give 128 mg of amide 24 (75% yield)

### (Analytical value of the desired reaction product)

¹H-NMR (DMSO-d6):8.2 (1H, br.t), 8.0 (1H, s), 7.95 (1H, s), 7.75 (2H, t), 7.3-7.7 (12H, m), 5.05 (1H, d), 4.55-4.65 (2H, m), 4.3-4.4 (3H, m), 4.1 (1H, m), 3.64 (1H, d), 3.4-3.5 (4H, m), 3.2-3.3 (2H, m), 3.16 (4H, d), 2.95-3.1 (4H, br.t), 2.8-2.9 (4H, m), 2.5-2.6 (1H, m), 2.2-2.35 (4H, t), 2.0-2.1 (4H, t), 1.8-2.0 (1H, m), 1.2-1.7 (24H, m), 1.4 (18H, s)
HPLC Retention Time (Analytical Condition A); 14.7 min (0.1% aqueous trifluoroacetic acid/acetonitrile = 85/15 (18 min) 5/95)

### EXAMPLE 1-2

### (Synthesis of Bis-iminobiotin Compound 26)

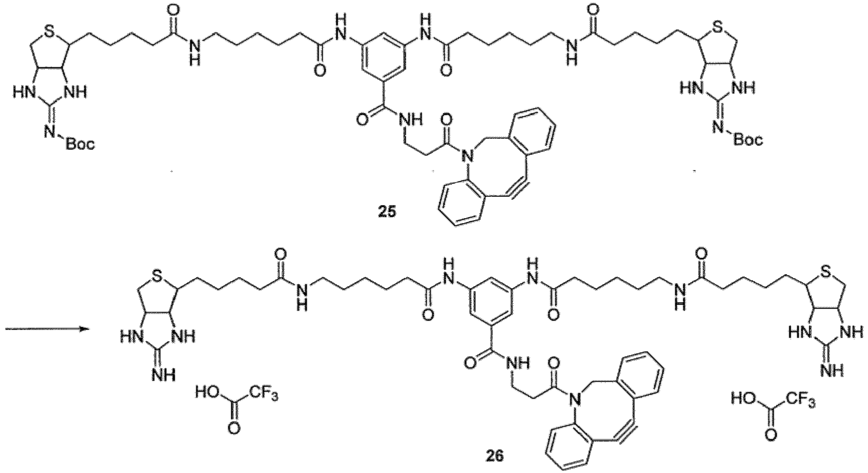

To 15 mg (11.6 µmol) of amide 25 synthesized in Example 1-1 was added 0.5 mL of trifluoroacetic acid and stirred at room temperature for 1 hours. The reaction was concentrated in vacuo at 60 °C to give 13.3 mg of a trifluoroacetate of bis-iminobiotin 26. (95% yield)

### (Analytical value of the desired reaction product)

¹H-NMR (DMSO-d6):10.0 (2H, s), 8.2 (1H, br.t), 8.0 (1H, s), 7.95 (1H, s), 7.75 (2H, t), 7.72-7.3 (15H, m), 5.05 (1H, d), 4.61-4.55 (2H, m), 4.47-4.40 (3H, m), 4.19 (1H, m), 3.60 (1H, d), 3.53-3.43 (4H, m), 3.34-3.22 (2H, m), 3.16 (4H, d), 3.11-2.89 (4H, br.t), 2.82-2.75 (4H, m), 2.61-2.57 (1H, m), 2.30 (4H, t), 2.05 (4H, t), 1.98-1.90 (1H, m), 1.72-1.27 (24H, m)
HPLC Retention Time (Analytical Condition A); 10.3 min (0.1% aqueous trifluoroacetic acid/acetonitrile = 85/15 (18 min) 5/95)

### EXAMPLE 1-3

### (Synthesis of Bis-iminobiotin-Fluorosein Compound 28)

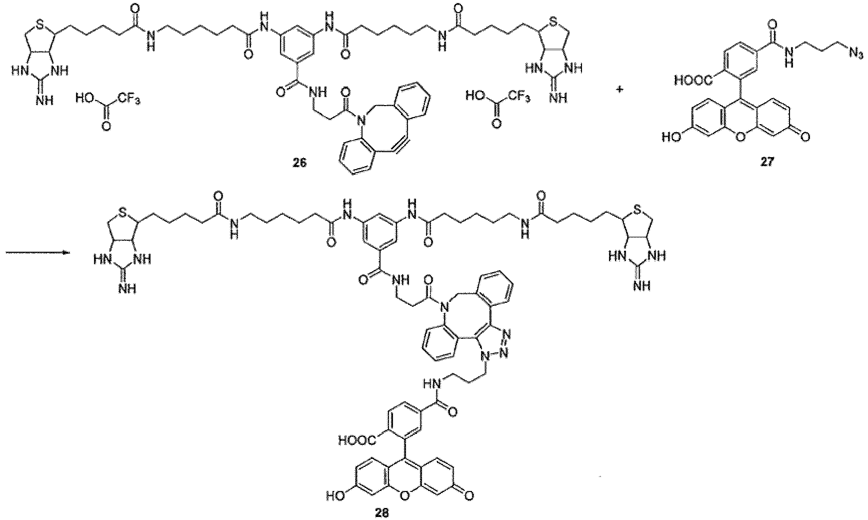

Trifluoroacetate of 13 mg (12 µmol) of bis-iminobiotin 26 synthesized in Example 1-2 was dissolved in 1mL of dehydrated dimethylformamide and 0.72 mL of chloroform solution was added. In addition, a solution of 11 mg (2 equiv) of 6-carboxyfluorocein azide 27 in chloroform was added. After stirring at room temperature for 1 night, the reaction was concentrated in vacuo at 60 °C The residue was washed twice with 2 mL of 1-specified hydrochloric acid aqueous solution, and then dried under reduced pressure to obtain 25 mg of bis-iminobiotin-fluorocein 28 in amorphous form.
¹H-NMR (DMSO-d6):10.4 (1H, br.s), 10.0 (2H, s), 8.3 (3H, m), 8.1 (1H, d), 8.0 (1H, s), 7.95 (1H, s), 7.75 (2H, t), 7.72-7.3 (15H, m), 7.53 (1H, d), 7.00 (1H, d), 6.46 (1H, d), 6.22 (3H, m), 6.11 (1H, d), 5.05 (2H, m), 4.61-4.55 (2H, m), 4.47-4.40 (3H, m), 4.19 (1H, m), 3.60 (1H, d), 3.53-3.43 (6H, m), 3.34-3.22 (2H, m), 3.16 (4H, d), 3.11-2.89 (4H, br.t), 2.82-2.75 (4H, m), 2.62-2.57 (3H, m), 2.30 (4H, t), 2.05 (4H, t), 1.98-1.90 (1H, m), 1.72-1.27 (24H, m)
HPLC Retention Time (Analytical Condition A); 12.6 min (0.1% aqueous trifluoroacetic acid/acetonitrile = 85/15 (18 min) 5/95)

### EXAMPLE 2-1

### (Synthesis of Bis(Boc-Iminobiotin) Compound 30)

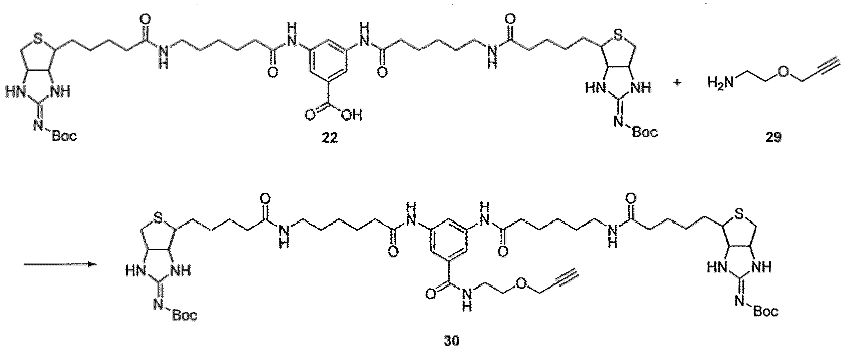

In Example 1-1, instead of using 135 mg of bis (Boc-iminobiotin) 22 and 36 mg of amine 23, 500 mg (0.49 mmol) of bis (Boc-iminobiotin) 22 and 36 mg (1.27 equivalents) of amine 29 were used, and the reaction was carried out according to the synthetic method described in Example 1-1 to obtain 128 mg of bis (Boc-iminobiotin) 30 (49% yield)

### (Analytical value of the desired reaction product)

¹H-NMR (DMSO-d6):9.97 (2H, s), 8.4 (1H, br.t), 8.0 (1H, s), 7.95 (1H, s), 7.74 (2H, t), 7.65 (3H, d), 4.6-4.5 (2H, m), 4.35-4.25 (2H, m), 4.16 (2H, d), 4.1 (1H, m), 3.56 (2H, t), 3.45-3.35 (3H, m), 3.25-3.15 (1H, m), 2.95-3.1 (4H, br.q), 2.9-2.75 (4H, m), 2.45-2.2 (4H, t), 2.1-2.0 (4H, t), 1.2-1.7 (24H, m), 1.4 (18H, s)
HPLC hold time (analytical condition A); 11.3 minutes (0.1% aqueous trifluoroacetic acid/acetonitrile = 85/15 (12 minutes) 5/95)

### EXAMPLE 2-2

### (Synthesis of Bis-iminobiotin Compound 31)

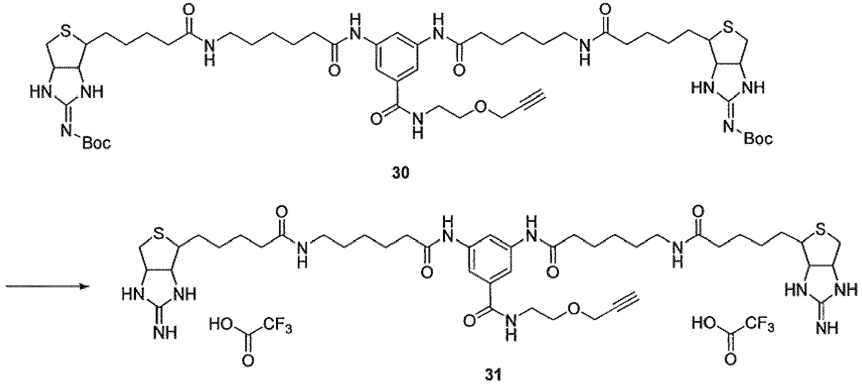

In Example 1-2, instead of using 15 mg (11.6 µmol) of amide 25, a 15 mg (13.5 µmol) of bis(Boc-iminobiotin)-acetylene 30 synthesized in Example 2-1 was used and the reaction was carried out according to the synthetic method described in Example 1-2 to obtain 13.8 mg of trifluoroacetate of bis-iminobiotin 31 (100% yield)

### (Analytical value of the desired reaction product)

¹H-NMR (DMSO-d6):9.99 (2H, s), 8.40 (1H, t), 8.35 (2H, s), 8.09 (1H, s), 7.79 (2H, t), 7.70 (4H, br.s), 7.66 (2H, s), 4.65-4.61 (2H, m), 4.47-4.42 (2H, m), 4.16 (2H, d), 3.56 (2H, t), 3.45-3.38 (3H, m), 3.27-3.20 (2H, m), 3.06 (4H, q), 2.94-2.73 (4H, m), 2.31 (4H, t), 2.06 (4H, t), 1.71-1.24 (24H, m)
HPLC Retention Time (Analytical Condition A); 9.68 min (0.1% aqueous trifluoroacetic acid/acetonitrile = 85/15 (12 min) 5/95)

### EXAMPLE 3-1

### (Synthesis of Bis (Boc-Iminobiotin) Compound 33)

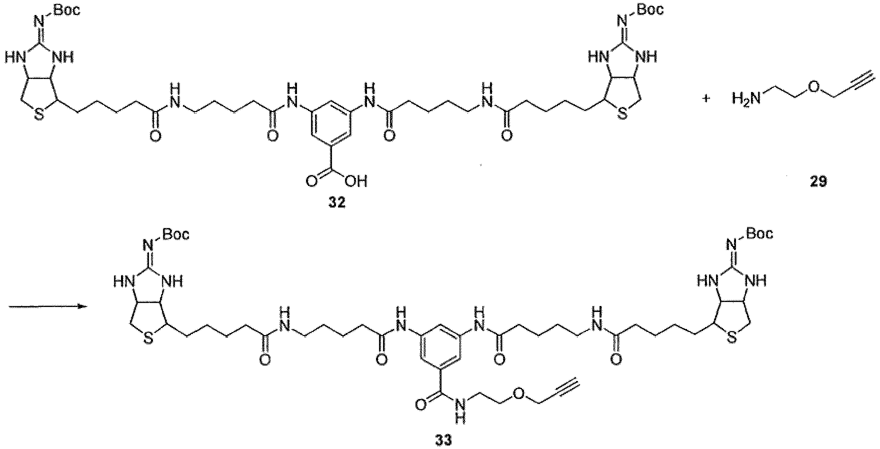

In Example 1-1, instead of using 135 mg of bis (Boc-iminobiotin) 22 and 36 mg of amine 23, 355mg (355 µmol) of bis (Boc-iminobiotin) 32 and 55.2 µL (1.5 equiv) of amine 29 were used, and the reaction was carried out according to the synthetic method described in Example 1-1 to obtain 55.6 mg of bis (Boc-iminobiotin) 33 as the target reaction product. (Yield 15%)

### (Analytical value of the desired reaction product)

¹H-NMR (DMSO-d6):9.99 (2H, s), 8.39 (1H, br.t), 8.07 (1H, s), 7.93 (2H, s), 7.77 (2H, t), 7.65 (4H, br.s), 4.53-4.84 (2H, m), 4.27-4.23 (2H, m), 4.17 (2H, d), 3.56 (2H, t), 3.44-3.38 (3H, m), 3.21-3.14 (2H, m), 3.09-3.01 (4H, br.q), 2.83-2.80 (4H, m), 2.31 (4H, t), 2.05 (4H, t), 1.62-1.42 (20H, m), 1.36 (18H, s)
HPLC retention times (analytical condition B); 4.99 min (0.1% aqueous trifluoroacetic acid/acetonitrile = 85/15 (7 min) 5/95)

### EXAMPLE 3-2

### (Synthesis of Bis-iminobiotin Compound 34)

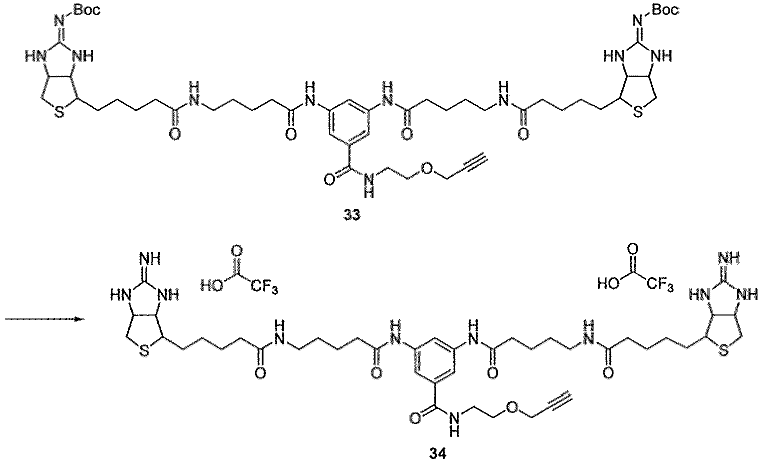

In Example 1-2, instead of using 15 mg (11.6 µmol) of amide 25, 5.1 mg (4.7 µmol) of bis (Boc-iminobiotin) 33 synthesized in Example 3-1 was used, and the reaction was carried out according to the synthetic method described in Example 1-2 to obtain 5.2 mg of trifluoroacetate of bis-iminobiotin 34 (100% yield)

### (Analytical value of the desired reaction product)

¹H-NMR (DMSO-d6):10.00 (2H, s), 8.40 (1H, t), 8.35 (2H, s), 8.07 (1H, s), 7.79 (2H, t), 7.70 (4H, br.s), 7.66 (2H, s), 4.66-4.61 (2H, m), 4.47-4.42 (2H, m), 4.16 (2H, d), 3.56 (2H, t), 3.45-3.39 (3H, m), 3.27-3.20 (2H, m), 3.05 (4H, q), 2.94-2.73 (4H, m), 2.31 (4H, t), 2.06 (4H, t), 1.71-1.24 (20H, m)
HPLC retention times (analytical condition B); 3.76 min (0.1% aqueous trifluoroacetic acid/acetonitrile = 85/15 (7 min) 5/95)

### EXAMPLE 4-1

### (Bis(Boc-Iminobiotin)-Synthesis of azide compound 36)

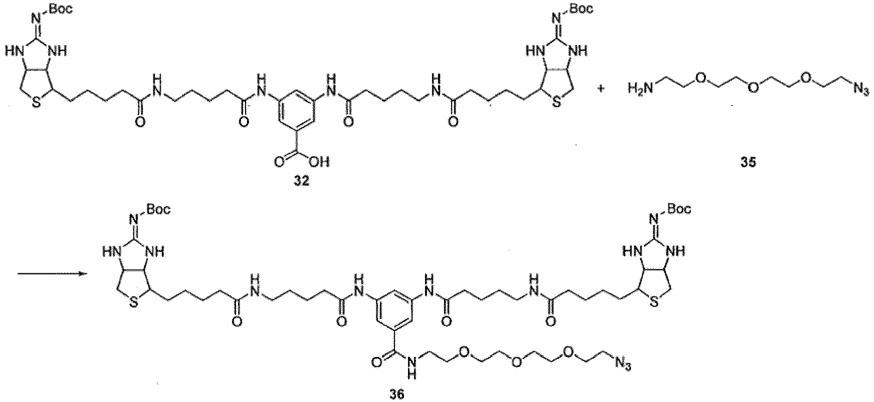

In Example 1-1, instead of using 135 mg of bis (Boc-iminobiotin) 22 and 36 mg of amine 23, 50 mg (50 µmol) of bis (Boc-iminobiotin) 32 and 15.0 µL (1.5 equiv) of amine 35 were used, and a reaction was carried out according to the synthetic method described in Example 1-1 using to obtain 16.0 mg of bis (Boc-iminobiotin)-azido 36 (Yield 27%)

### (Analytical value of the desired reaction product)

¹H-NMR (DMSO-d6):10.0 (2H, s), 8.35 (1H, t), 8.05 (1H, s), 7.93 (2H, s), 7.77 (2H, t), 7.66 (4H, m), 4.55 (2H, m), 4.27 (2H, m), 3.54-3.51 (12H, m), 3.39-3.37 (2H, m), 3.18-3.16 (2H, m), 3.06-3.04 (4H, m), 2.83-2.75 (4H, m), 2.30 (4H, t), 2.05 (4H, t), 1.70-1.41 (22H, m), 1.35 (18H, s)
HPLC retention times (analytical condition B); 5.64 min (0.1% aqueous trifluoroacetic acid/acetonitrile = 85/15 (7 min) 20/80

### EXAMPLE 4-2

### (Synthesis of Bis-iminobiotin-azide Compound 37)

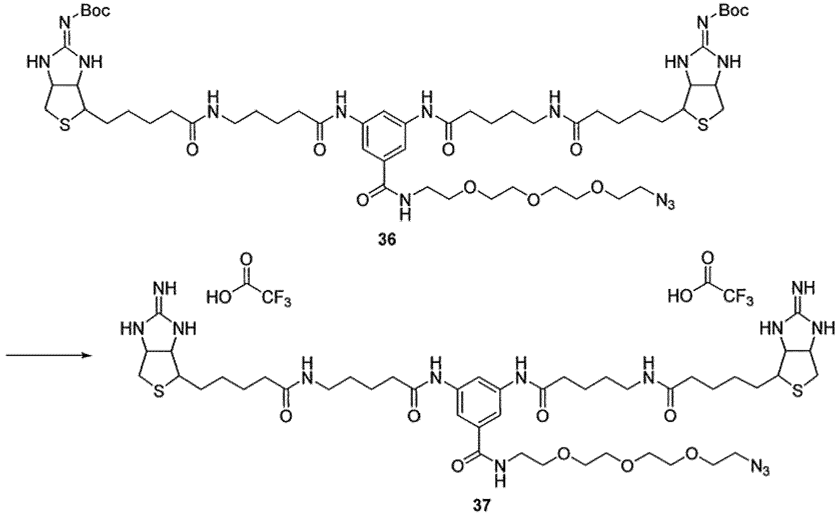

In Example 1-2, instead of using 15 mg (11.6 µmol) of amide 25, 16 mg (13 µmol) of bis(Boc-iminobiotin)-azide 36 synthesized in Example 4-1 was used, and a reaction was carried out according to the synthetic method described in Example 1-2 to obtain 14.5 mg of a trifluoroacetate of bis-iminobiotin-azide 37 (100% yield)

### (Analytical value of the desired reaction product)

¹H-NMR (DMSO-d6):10.00 (2H, s), 8.47-8.44 (1H, m), 8.35 (1H, m), 8.21 (2H, m), 8.05 (1H, m), 7.81-7.76 (5H, m), 7.67 (2H, d), 4.66-4.61 (2H, m), 4.47-4.42 (2H, m), 3.60-3.50 (12H, m), 3.57 (2H, m), 3.25 (2H, m), 3.05 (4H, q), 2.94-2.75 (4H, m), 2.31 (4H, t), 2.06 (4H, t), 1.71-1.24 (22H, m)
HPLC retention times (analytical condition B); 4.34 min (0.1% aqueous trifluoroacetic acid/acetonitrile = 85/15 (7 min) 20/80

## Claims

1. A substrate for drug delivery to a substance labeled with streptavidin, **characterized in that** comprises a bis-iminobiotin compound represented by the following general formula 9: 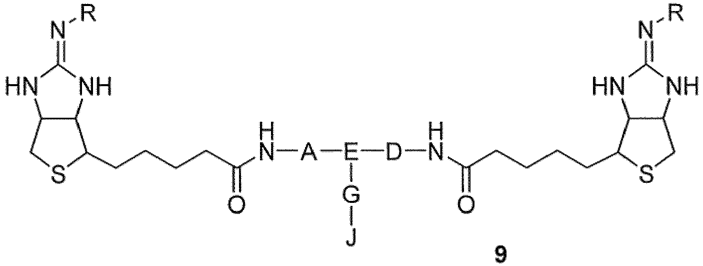 (In the formula, A, D, and E are spacers connecting 2two bicyclo rings, and E represents a structure capable of taking a branch, and may each have a substituent, and may form a ring structure. J represents a functional group for performing a click reaction, G represents a spacer for bonding E and J, and R represents a hydrogen, an acetyl group, a benzyl group, a trifluoroacetyl group or a Boc group.),
provided that the following compounds 10-24 and 10-25 are excludes:

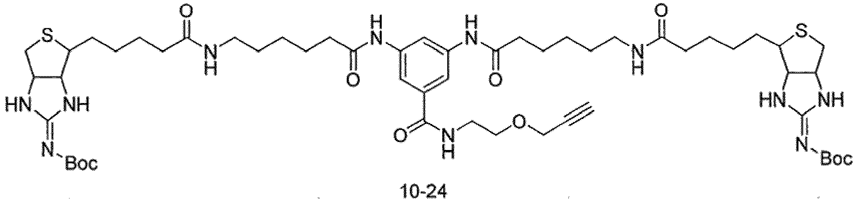

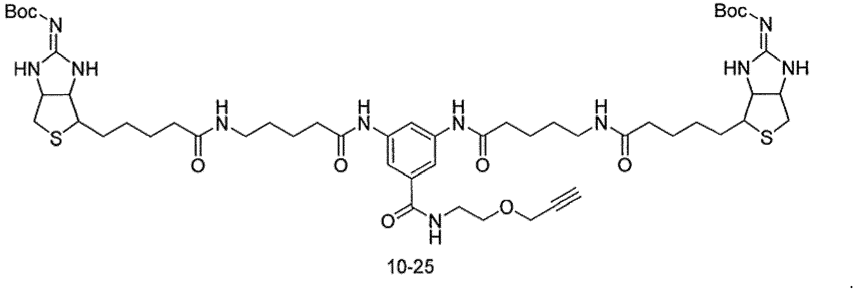

2. The substrate for drug delivery according to claim 1, wherein in general formula 9,
RisH,
A and D are one of the four structures in the following table,
| | A, D | |
|---|---|---|
| Iminobiotin side | -(CH2)4- | E side |
| | -(CH2)5- | |
| | -(CH2)6- | |
| | -(CH2)7- | |
| | -CH(COOH)CH2CH2CH2CH2- | -CH(COOH)CH2CH2CH2CH2- |
| | -CH(COOH)CH2CH2CH2- | |
E is any one of the following three structures (where (A) represents a bond to A, (D) represents a bond to D, and (G) represents a bond to G): 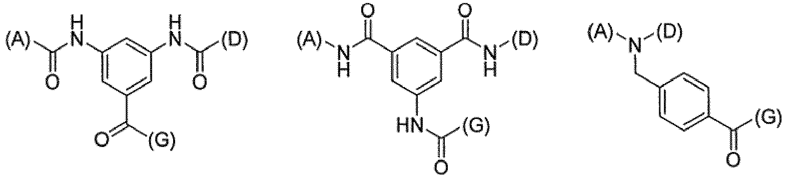 G is any one of the six structures in the following table,
| | G | |
|---|---|---|
| E side | -NHCH2CH2OCH2- | J side |
| | -NH(CH2CH2O)3CH2- | |
| | -NH(CH2CH2O)3CH2CH2CONHCH2- | |
| | -NH(CH2CH2O)3CH2CH2COOCH2- | |
| | -NHCH2CH2CO- | |
| | -CH2O(CH2CH2O)2CH2CH2- | |
J is any one of the following three structures (where (G) represents a bond to G):

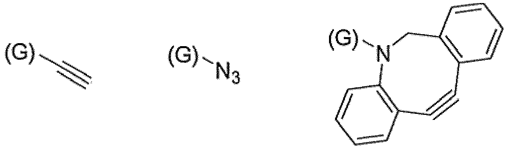

3. The substrate for drug delivery according to claim 2, wherein E is the following structure (where (A) represents binding to A, (D) represents binding to D, and (G) represents binding to G):

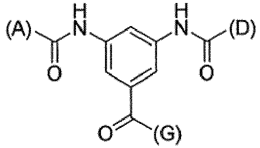

4. The substrate for drug delivery of claim 2, wherein the bis-iminobiotin compound is one selected from the following compounds 13-1 to 13-23:

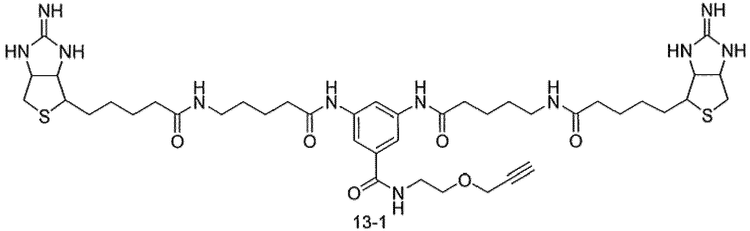

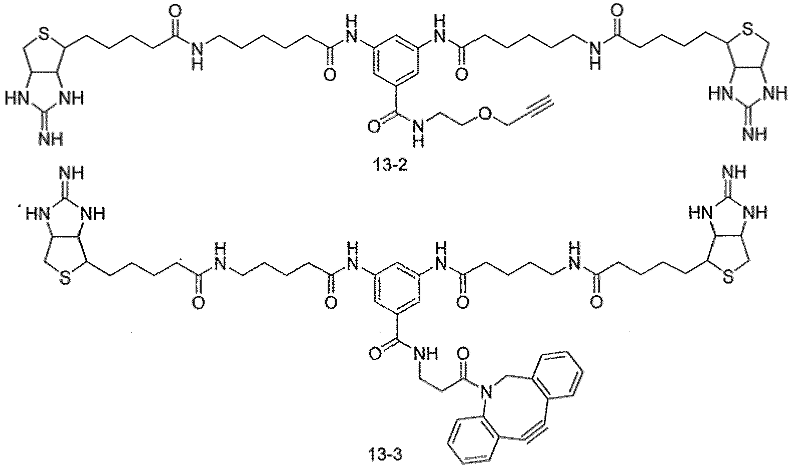

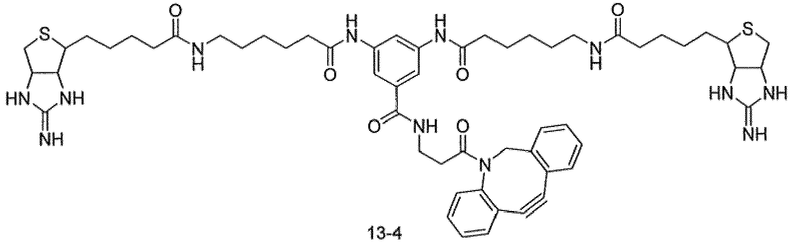

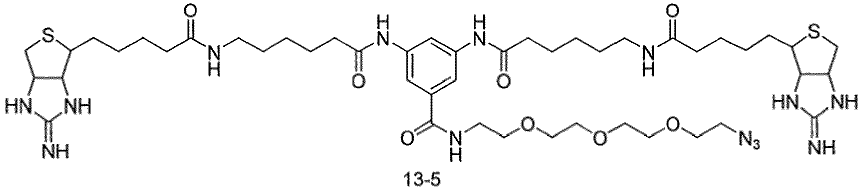

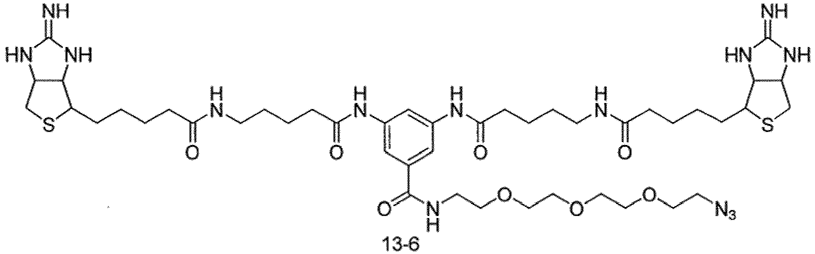

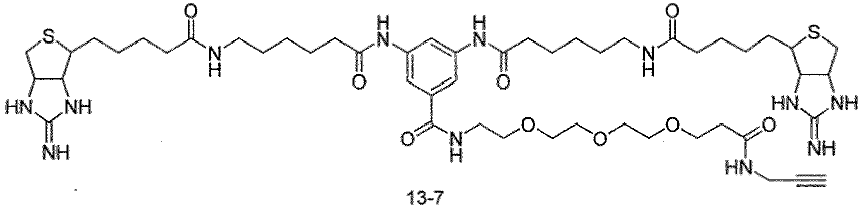

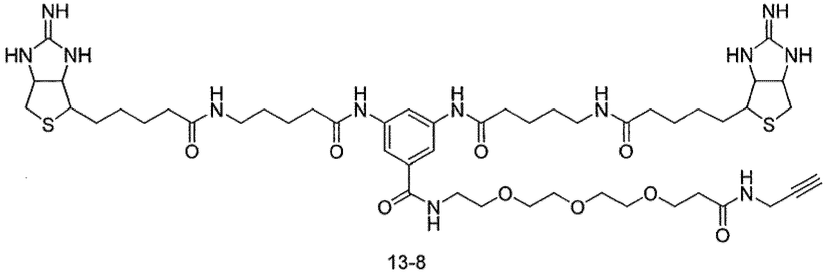

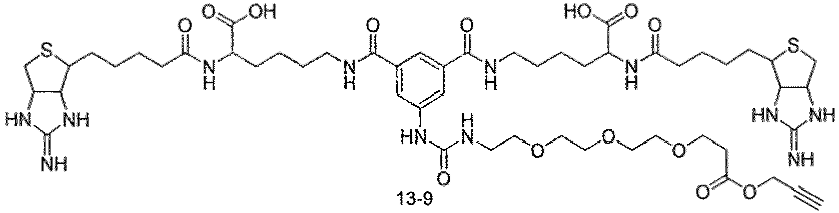

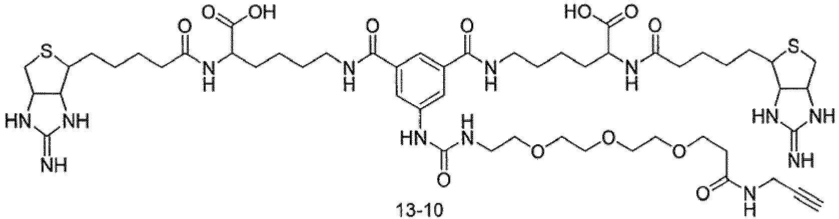

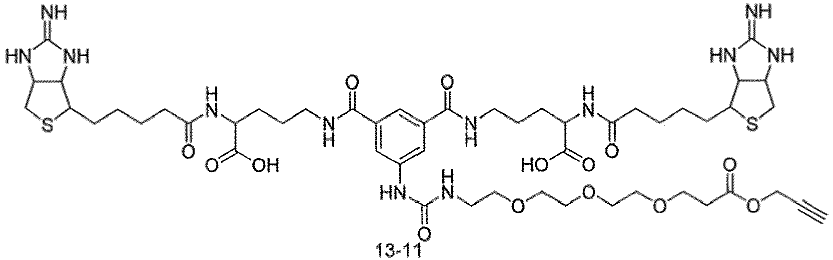

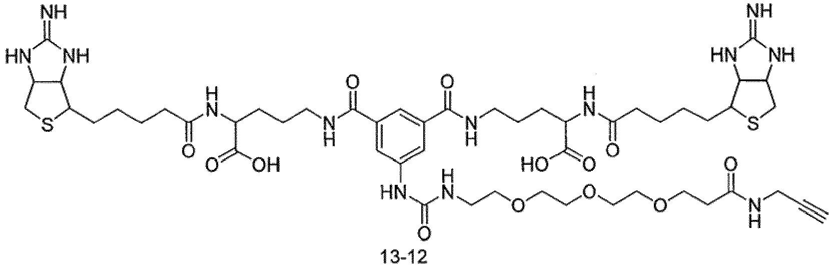

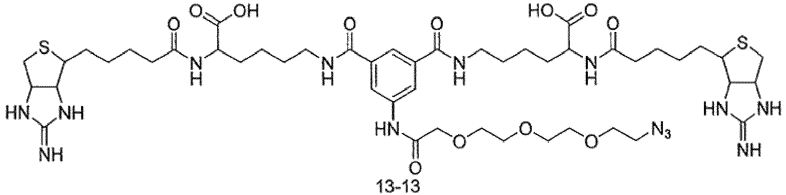

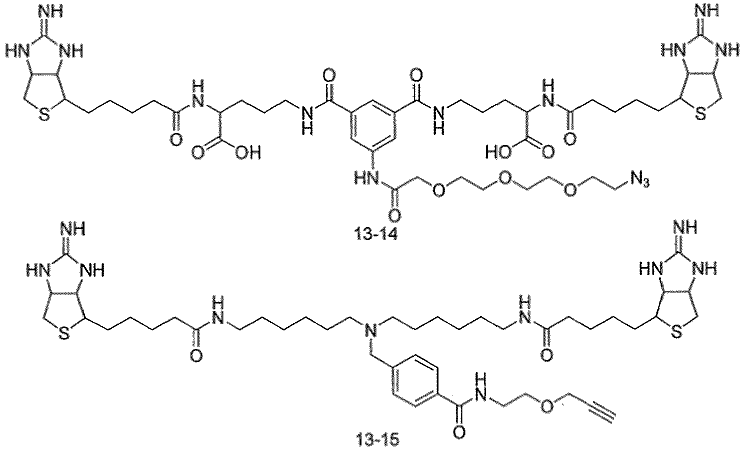

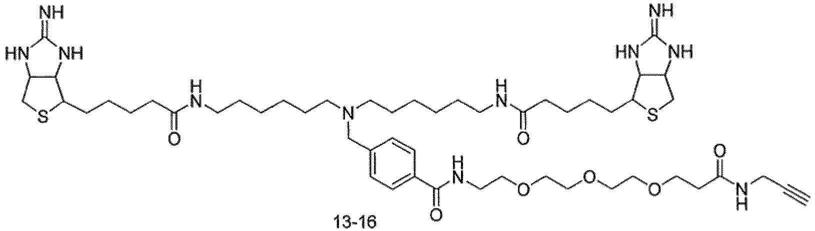

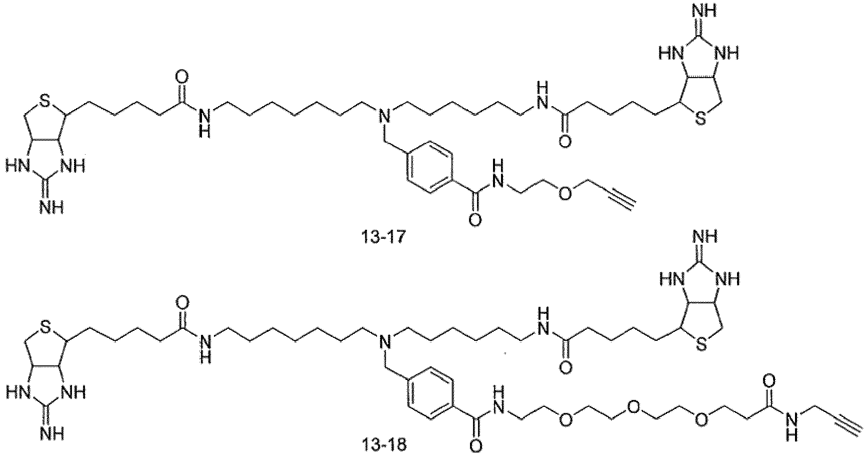

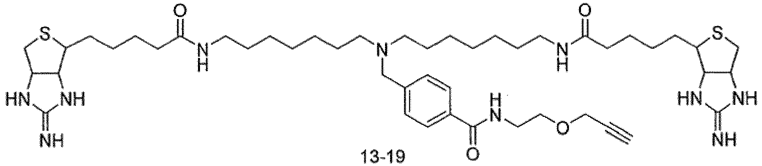

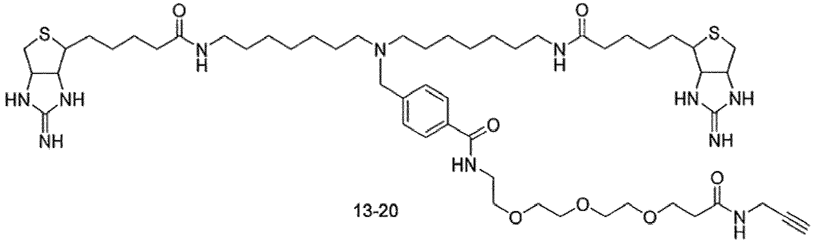

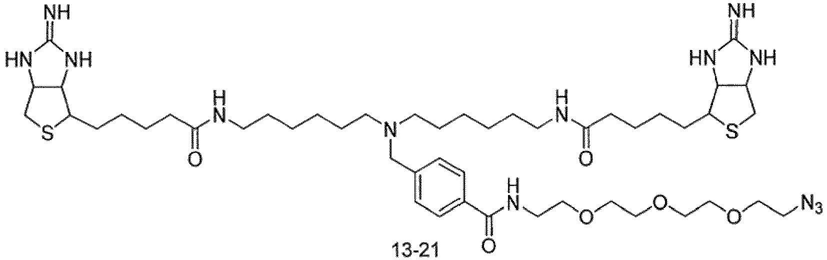

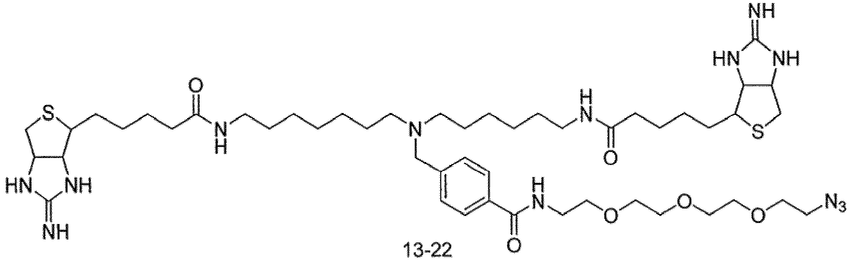

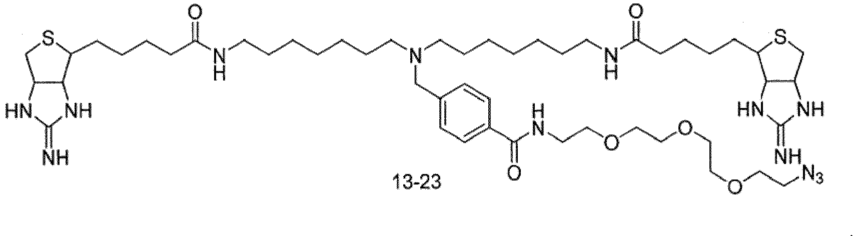

5. The substrate for drug delivery of claim 4, wherein the bisimino biotin compound is any one of the following compounds:

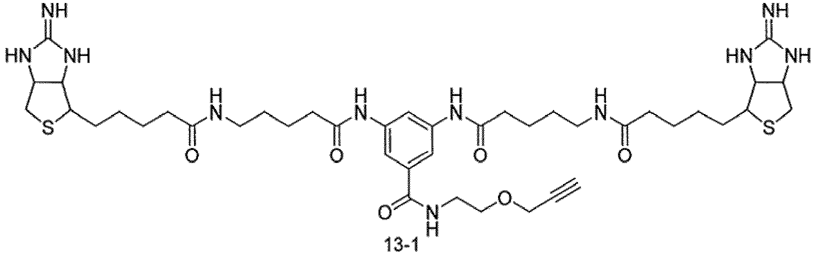

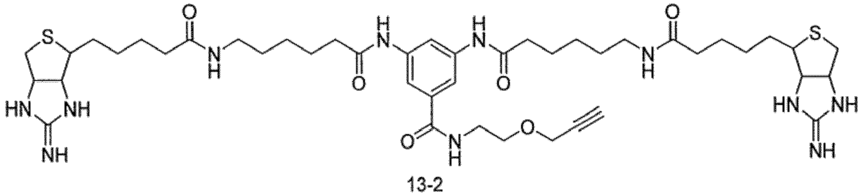

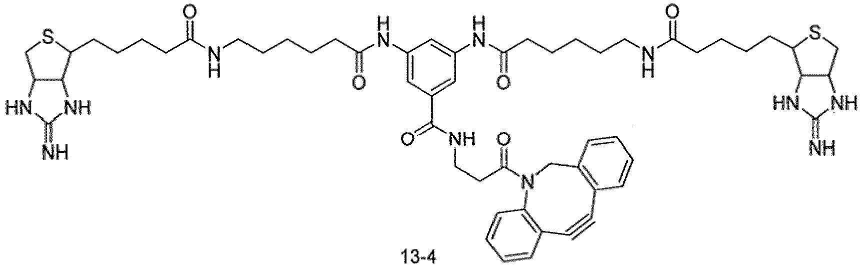

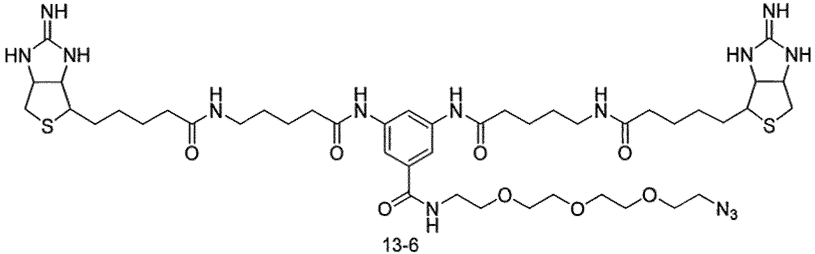

6. A drug delivery agent for drug delivery to a substance labeled with streptavidin, comprising a drug-conjugated bis-iminobiotin compound, represented by the general formula 14: 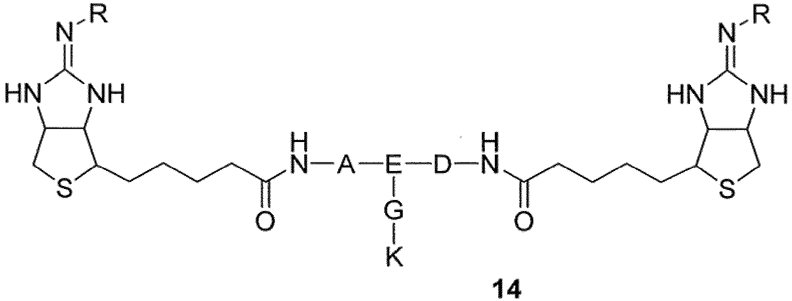 (In the formula,
A, D, and E are spacers that bind two bicyclic rings, and G represents a spacer that binds E and K,
A and D are one of the four structures in the following table,
**Table 3**
| | A, D | |
|---|---|---|
| Iminobiotin side | -(CH2)4- | E side |
| | -(CH2)5- | |
| | -(CH2)6- | |
| | -(CH2)7- | |
| | -CH(COOH)CH2CH2CH2CH2- | |
| | -CH(COOH)CH2CH2CH2- | |
E is any one of the following three structures (where (A) represents a bond to A, (D) represents a bond to D, and (G) represents a bond to G): 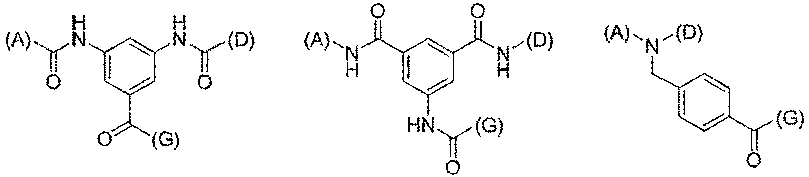
G is any one of the six structures in the following table,
**Table 4**
| | G | |
|---|---|---|
| E side | -NHCH2CH2OCH2- | J side |
| | -NH(CH2CH2O)3CH2- | |
| | -NH(CH2CH2O)3CH2CH2CONHCH2- | |
| | -NH(CH2CH2O)3CH2CH2COOCH2- | |
| | -NHCH2CH2CO- | |
| | -CH2O(CH2CH2O)2CH2CH2- | |
R is H,
K represents a drug.).

7. Use of a bis-iminobiotin compound according to any one of claims 1 to 5 as a substrate for drug delivery to a substance labeled with streptavidin.

8. Use of the drug-conjugated bis-iminobiotin compound of claim 6 as a drug delivery agent for drug delivery to a substance labeled with streptavidin.

9. A method of delivering a drug to a streptavidin-labeled substance comprising:
binding a drug to a substrate for drug delivery; binding a substrate for drug delivery to said streptavidin-labeled substance via said substrate for drug delivery; and binding said drug to said streptavidin-labeled substance,
wherein the substrate for drug delivery is a bis-iminobiotin compound according to any one of claims 1 to 5.

10. A method for producing a drug delivery agent for drug delivery to a substance labeled with streptavidin, comprising binding a drug utilizing a functional group J to a bis-iminobiotin compound according to any one of claims 1 to 5.

11. The method for producing a drug delivery agent according to claim 10, wherein the drug is a bioactive substance or a fluorescent compound.

12. Use of a bis-iminobiotin compound according to any one of claims 1 to 5 in a method for producing a drug delivery agent for drug delivery to a substance labeled with streptavidin.

13. The use of claim 12, wherein said drug is a bioactive substance or a fluorescent compound.
